(19) Europäisches Patentamt
European Patent Office
Office européen des brevets

(11) **EP 4 242 303 A1**

(12) **EUROPEAN PATENT APPLICATION**

(43) Date of publication:
**13.09.2023 Bulletin 2023/37**

(21) Application number: **22160942.3**

(22) Date of filing: **08.03.2022**

(51) International Patent Classification (IPC):
***C12N 9/80*** *(2006.01)*

(52) Cooperative Patent Classification (CPC):
**C12N 9/80**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Novozymes A/S**
**2880 Bagsvaerd (DK)**

(72) Inventor: **Jens, Magnus, Ekloef**
**2880 Bagsvaerd (DK)**

(74) Representative: **NZ EPO Representatives**
**Krogshoejvej 36**
**2880 Bagsvaerd (DK)**

Remarks:
The complete document including Reference Table(s) and the Sequence Listing(s) can be downloaded from the EPO website

(54) **FUSION POLYPEPTIDES WITH DEAMIDASE INHIBITOR AND DEAMIDASE DOMAINS**

(57)    The present invention relates to polypeptides having deamidase activity and polynucleotides encoding the polypeptides. The invention also relates to nucleic acid constructs, vectors, and host cells comprising the polynucleotides as well as methods of producing and using the polypeptides.

EP 4 242 303 A1

## Description

### Reference to a Sequence Listing

This application contains a Sequence Listing in computer readable form, which is incorporated herein by reference.

### FIELD OF THE INVENTION

The present invention relates to fusion polypeptides comprising propeptides and polypeptides having deamidase activity, as well as the separate propeptides and polypeptides having deamidase activity; polynucleotides encoding the polypeptides, nucleic acid constructs, vectors, and host cells comprising the polynucleotides as well as methods of producing and using the polypeptides.

### BACKGROUND

Deamidase enzymes are produced by microbial cells in an inactive proform, which comprises a propeptide domain tightly bound to a deamidase domain. The proform is expressed as a fusion protein, which has reduced deamidase activity to protect the viability of the host cell. In nature, the fusion protein is post-processed to remove the propeptide and release the active deamidase outside of the host cell. However, in recombinant expression systems, the fusion protein is secreted outside of the host cell as an inactive proform comprising the propeptide. For many deamidase proforms, the propeptide domain cannot be separated from the deamidase domain by simple cleavage because of the high binding affinity of the propeptide towards the deamidase polypeptide. For other deamidase proforms produced in recombinant expression systems, the binding affinitiy between the propeptide and the deamidase polypeptide is too weak, resulting in an activated deamidase which reduces viability of the recombinant host cell and makes commercial production impossible.

The object of the present invention is to provide new proforms of deamidase enzymes (fusion polypeptides), where the binding affinity of the propeptide domain to the mature deamidase domain has been adjusted to allow recombinant expression and secretion without reducing viability of the host cell, and at the same time allowing extracellular separation of the propeptide and the active mature deamidase enzyme.

### SUMMARY OF THE INVENTION

The present invention provides fusion polypeptides comprising propeptides and deamidase polypeptides, as well as the separate propeptides and deamidase polypeptides, and the polynucleotides encoding the peptides and polypeptides.

Accordingly, in a first aspect, the present invention relates to recombinant fusion polypeptides comprising or consisting of

(a) a first polypeptide comprising a deamidase inhibitory domain, and
(b) a second polypeptide having deamidase activity;

wherein the fusion polypeptide has a thermal unfolding temperature in the range of 65-82°C; and the fusion polypeptide has less than 50% deamidase activity relative to the deamidase activity of the second polypeptide.

In another aspect, the invention provides a method for producing a polypeptide having deamidase activity, comprising contacting the fusion polypeptide of the invention with an endopeptidase to separate the first polypeptide from the second polypeptide.

In yet another aspect, the invention provides a composition exhibiting deamidase activity, comprising the separated first polypeptide and second polypeptide of the fusion polypeptide of the invention.

The present invention also relates to polynucleotides encoding the polypeptides of the present invention; nucleic acid constructs; recombinant expression vectors; recombinant host cells comprising the polynucleotides; and methods of producing the polypeptides.

Other aspects and embodiments of the invention are apparent from the description and examples.

### Sequences

SEQ ID NO: 1: Polynucleotide encoding a propeptide from *Chryseobacterium* sp-62563.
SEQ ID NO: 2: Amino acid sequence of propeptide encoded by SEQ ID NO: 1.
SEQ ID NO: 3: Polynucleotide encoding a deamidase polypeptide from *Chryseobacterium* sp-62563.
SEQ ID NO: 4: Amino acid sequence of deamidase polypeptide encoded by SEQ ID NO: 3.
SEQ ID NO: 5: Amino acid sequence of a fusion polypeptide comprising SEQ ID NOs: 2 and 4.
SEQ ID NO: 6: Polynucleotide encoding a propeptide from *Chryseobacterium gambrini*.
SEQ ID NO: 7: Amino acid sequence of propeptide encoded by SEQ ID NO: 6.
SEQ ID NO: 8: Polynucleotide encoding a deamidase polypeptide from *Chryseobacterium gambrini*.
SEQ ID NO: 9: Amino acid sequence of deamidase polypeptide encoded by SEQ ID NO: 8.
SEQ ID NO: 10: Amino acid sequence of a fusion polypeptide comprising SEQ ID NOs: 7 and 9.
SEQ ID NO: 11: Polynucleotide encoding a propeptide from *Chryseobacterium culicis.*
SEQ ID NO: 12: Amino acid sequence of propeptide encoded by SEQ ID NO: 11.
SEQ ID NO: 13: Polynucleotide encoding a deamidase polypeptide from *Chryseobacterium culicis.*
SEQ ID NO: 14: Amino acid sequence of deamidase polypeptide encoded by SEQ ID NO: 13.
SEQ ID NO: 15: Amino acid sequence of a fusion polypeptide comprising SEQ ID NOs: 12 and 14.
SEQ ID NO: 16: Polynucleotide encoding a propeptide from *Chryseobacterium defluvii*.
SEQ ID NO: 17: Amino acid sequence of propeptide encoded by SEQ ID NO: 16.
SEQ ID NO: 18: Polynucleotide encoding a deamidase polypeptide from *Chryseobacterium defluvii.*
SEQ ID NO: 19: Amino acid sequence of deamidase polypeptide encoded by SEQ ID NO: 18.
SEQ ID NO: 20: Amino acid sequence of a fusion polypeptide comprising SEQ ID NOs: 17 and 19.
SEQ ID NO: 21: Amino acid sequence motif overlapping with the deamidase active site.
SEQ ID NO: 22: Amino acid sequence motif overlapping with the deamidase active site.
SEQ ID NO: 23: Amino acid sequence motif overlapping with the deamidase active site.
SEQ ID NO: 24: Amino acid sequence motif covering the deamidase inhibitory domain.
SEQ ID NO: 25: Amino acid sequence motif covering the deamidase inhibitory domain.

**Definitions**

In accordance with this detailed description, the following definitions apply. Note that the singular forms "a," "an," and "the" include plural references unless the context clearly dictates otherwise.

Unless defined otherwise or clearly indicated by context, all technical and scientific terms used herein have the same meaning as commonly understood by one of ordinary skill in the art to which this invention belongs.

**Deamidase:** The term "deamidase" means a protein-glutamine glutaminase (also known as glutaminylpeptide glutaminase) activity, as described in EC 3.5.1.44, which catalyzes the hydrolysis of the gamma-amide of glutamine substituted at the carboxyl position or both the alpha-amino and carboxyl positions, e.g., L-glutaminylglycine and L-phenylalanyl-L-glutaminylglycine. Thus, deamidases can deamidate glutamine residues in proteins to glutamate residues and are also referred to as protein glutamine deamidase. Deamidases comprise a Cys-His-Asp catalytic triad (e.g., Cys-156, His-197, and Asp-217, as shown in Hashizume et al. "Crystal structures of protein glutaminase and its pro forms converted into enzyme-substrate complex", Journal of Biological Chemistry, vol. 286, no. 44, pp. 38691-38702) and belong to the InterPro entry IPR041325. In a preferred embodiment, the deamidases of the present invention belong to PFAM domain PF18626. The deamidase amino acid sequence may comprise the amino acid sequence motif(s) DGCYARAH (SEQ ID NO: 21), CYARAH[R/K/Q] (SEQ ID NO: 22), and/or HVA[L/V/I]LVS (SEQ ID NO: 23), which overlap the active site.

**Deamidase Activity:** Deamidase activity was measured, as described in Example 1, by using a fluorescence substrate comprising a glutamine residue and a fluorescence quenching group. The glutamine residue is converted to a glutamate residue by the deamidase activity, and the substrate is then cleaved by a glutamyl endopeptidase to remove the fluorescence quenching group.

**Deamidase Inhibitory Domain:** The term "deamidase inhibitory domain" means a sequence of amino acids that interacts with the amino acid residues of the deamidase active site and inhibits or reduces the deamidase activity. For example, the deamidase activity can be reduced to less than 50%, preferably less than 40%, in the presence of the deamidase inhibitory domain (as compared to the deamidase activity without the presence of the deamidase inhibitory activity). The deamidase inhibitory domain may comprise the amino acid sequence motif [I/M][L/I/V][S/T]AQ (SEQ ID NO: 24); which corresponds to amino acids 35-43 of SEQ ID NO: 5, or amino acids 39-43 of SEQ ID NO: 10, amino acids 39-43 of SEQ ID NO: 15, or amino acids 41-45 of SEQ ID NO: 20. The deamidase inhibitory domain may comprise the amino acid sequence motif [K/R][V/I/L][S/A/N]X[I/M][L/I/V][S/T]AQ (SEQ ID NO: 25); which corresponds to amino acids 35-43 of SEQ ID NO: 5, or amino acids 35-43 of SEQ ID NO: 10, amino acids 35-43 of SEQ ID NO: 15, or amino acids 37-45 of SEQ ID NO: 20.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Coding sequence:** The term "coding sequence" means a polynucleotide, which directly specifies the amino acid sequence of a polypeptide. The boundaries of the coding sequence are generally determined by an open reading frame, which begins with a start codon, such as ATG, GTG, or TTG, and ends with a stop codon, such as TAA, TAG, or TGA. The coding sequence may be a genomic DNA, cDNA, synthetic DNA, or a combination thereof.

**Control sequences:** The term "control sequences" means nucleic acid sequences involved in regulation of expression of a polynucleotide in a specific organism or in vitro. Each control sequence may be native (i.e., from the same gene) or heterologous (i.e., from a different gene) to the polynucleotide encoding the polypeptide, and native or heterologous to each other. Such control sequences include, but are not limited to leader, polyadenylation, prepropeptide, propeptide, signal peptide, promoter, terminator, enhancer, and transcription or translation initiator and terminator sequences. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Expression:** The term "expression" means any step involved in the production of a polypeptide including, but not limited to, transcription, post-transcriptional modification, translation, post-translational modification, and secretion.

**Expression vector:** An "expression vector" refers to a linear or circular DNA construct comprising a DNA sequence encoding a polypeptide, which coding sequence is operably linked to a suitable control sequence capable of effecting expression of the DNA in a suitable host. Such control sequences may include a promoter to effect transcription, an optional operator sequence to control transcription, a sequence encoding suitable ribosome binding sites on the mRNA, enhancers and sequences which control termination of transcription and translation.

**Extension:** The term "extension" means an addition of one or more amino acids to the amino and/or carboxyl terminus of a polypeptide, wherein the "extended" polypeptide has deamidase activity.

**Fragment:** The term "fragment" means a polypeptide having one or more amino acids absent from the amino and/or carboxyl terminus of the mature polypeptide, wherein the fragment has deamidase activity.

**Fusion polypeptide:** The term "fusion polypeptide" is a polypeptide in which one polypeptide of the invention is fused at the N-terminus and/or the C-terminus of another polypeptide of the invention. A fusion polypeptide is produced by fusing two or more polynucleotides encoding the polypeptides of the invention together. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779). A fusion polypeptide can further comprise a cleavage site between the two polypeptides. Upon secretion of the fusion protein. The fusion polypeptides may comprise a cleavage site for a site-specific endopeptidase, for example within 20 amino acids, preferably within 10 amino acids, of the C-terminal end of the first polypeptide. Examples of well-known site-specific endopeptidases include glutamyl endopeptidase (*e.g.*, EC 3.4.21.19 or EC 3.4.21.82), trypsin- and chymotrypsin-like endopeptidases (incl. enteropeptidase). Many other examples of cleavage sites and the corresponding endopeptidases include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

**Heterologous:** The term "heterologous" means, with respect to a host cell, that a polypeptide or nucleic acid does not naturally occur in the host cell. The term "heterologous" means, with respect to a polypeptide or nucleic acid, that a control sequence, e.g., promoter, of a polypeptide or nucleic acid is not naturally associated with the polypeptide or nucleic acid, i.e., the control sequence is from a gene other than the gene encoding the mature polypeptide.

**Host Strain or Host Cell:** A "host strain" or "host cell" is an organism into which an expression vector, phage, virus, or other DNA construct, including a polynucleotide encoding a polypeptide of interest (e.g., an amylase) has been introduced. Exemplary host strains are microorganism cells (e.g., bacteria, filamentous fungi, and yeast) capable of expressing the polypeptide of interest and/or fermenting saccharides. The term "host cell" includes protoplasts created from cells.

**Introduced:** The term "introduced" in the context of inserting a nucleic acid sequence into a cell, means "transfection", "transformation" or "transduction," as known in the art.

**Isolated:** The term "isolated" means a polypeptide, nucleic acid, cell, or other specified material or component that has been separated from at least one other material or component, including but not limited to, other proteins, nucleic acids, cells, etc. An isolated polypeptide, nucleic acid, cell or other material is thus in a form that does not occur in nature. An isolated polypeptide includes, but is not limited to, a culture broth containing the secreted polypeptide expressed in a host cell.

**Mature polypeptide:** The term "mature polypeptide" means a polypeptide in its mature form following N-terminal and/or C-terminal processing (e.g., removal of signal peptide). In one aspect, the mature polypeptide is SEQ ID NO: 4.

**Mature polypeptide coding sequence:** The term "mature polypeptide coding sequence" means a polynucleotide that encodes a mature polypeptide having deamidase activity. In one aspect, the mature polypeptide coding sequence is SEQ ID NO: 3.

**Native:** The term "native" means a nucleic acid or polypeptide naturally occurring in a host cell.

**Nucleic acid:** The term "nucleic acid" encompasses DNA, RNA, heteroduplexes, and synthetic molecules capable of encoding a polypeptide. Nucleic acids may be single stranded or double stranded, and may be chemical modifications. The terms "nucleic acid" and "polynucleotide" are used interchangeably. Because the genetic code is degenerate, more than one codon may be used to encode a particular amino acid, and the present compositions and methods encompass nucleotide sequences that encode a particular amino acid sequence. Unless otherwise indicated, nucleic acid sequences are presented in 5'-to-3' orientation.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic, and which comprises one or more control sequences operably linked to the nucleic acid sequence.

**Operably linked:** The term "operably linked" means that specified components are in a relationship (including but not limited to juxtaposition) permitting them to function in an intended manner. For example, a regulatory sequence is operably linked to a coding sequence such that expression of the coding sequence is under control of the regulatory sequence.

**Purified:** The term "purified" means a nucleic acid, polypeptide or cell that is substantially free from other components as determined by analytical techniques well known in the art (e.g., a purified polypeptide or nucleic acid may form a discrete band in an electrophoretic gel, chromatographic eluate, and/or a media subjected to density gradient centrifugation). A purified nucleic acid or polypeptide is at least about 50% pure, usually at least about 60%, about 65%, about 70%, about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure (e.g., percent by weight or on a molar basis). In a related sense, a composition is enriched for a molecule when there is a substantial increase in the concentration of the molecule after application of a purification or enrichment technique. The term "enriched" refers to a compound, polypeptide, cell, nucleic acid, amino acid, or other specified material or component that is present in a composition at a relative or absolute concentration that is higher than a starting composition.

In one aspect, the term "purified" as used herein refers to the polypeptide or cell being essentially free from components (especially insoluble components) from the production organism. In other aspects, the term "purified" refers to the polypeptide being essentially free of insoluble components (especially insoluble components) from the native organism from which it is obtained. In one aspect, the polypeptide is separated from some of the soluble components of the organism and culture medium from which it is recovered. The polypeptide may be purified (i.e., separated) by one or more of the unit operations filtration, precipitation, or chromatography.

Accordingly, the polypeptide may be purified such that only minor amounts of other proteins, in particular, other polypeptides, are present. The term "purified" as used herein may refer to removal of other components, particularly other proteins and most particularly other enzymes present in the cell of origin of the polypeptide. The polypeptide may be "substantially pure", i.e., free from other components from the organism in which it is produced, e.g., a host organism for recombinantly produced polypeptide. In one aspect, the polypeptide is at least 40% pure by weight of the total polypeptide material present in the preparation. In one aspect, the polypeptide is at least 50%, 60%, 70%, 80% or 90% pure by weight of the total polypeptide material present in the preparation. As used herein, a "substantially pure polypeptide" may denote a polypeptide preparation that contains at most 10%, preferably at most 8%, more preferably at most 6%, more preferably at most 5%, more preferably at most 4%, more preferably at most 3%, even more preferably at most 2%, most preferably at most 1%, and even most preferably at most 0.5% by weight of other polypeptide material with which the polypeptide is natively or recombinantly associated.

It is, therefore, preferred that the substantially pure polypeptide is at least 92% pure, preferably at least 94% pure, more preferably at least 95% pure, more preferably at least 96% pure, more preferably at least 97% pure, more preferably at least 98% pure, even more preferably at least 99% pure, most preferably at least 99.5% pure by weight of the total polypeptide material present in the preparation. The polypeptide of the present invention is preferably in a substantially pure form (i.e., the preparation is essentially free of other polypeptide material with which it is natively or recombinantly associated). This can be accomplished, for example by preparing the polypeptide by well-known recombinant methods or by classical purification methods.

**Recombinant:** The term "recombinant" is used in its conventional meaning to refer to the manipulation, *e.g.*, cutting and rejoining, of nucleic acid sequences to form constellations different from those found in nature. The term recombinant refers to a cell, nucleic acid, polypeptide or vector that has been modified from its native state. Thus, for example, recombinant cells express genes that are not found within the native (non-recombinant) form of the cell, or express native genes at different levels or under different conditions than found in nature. The term "recombinant" is synonymous with "genetically modified" and "transgenic".

**Recover:** The terms "recover" or "recovery" means the removal of a polypeptide from at least one fermentation broth component selected from the list of a cell, a nucleic acid, or other specified material, *e.g.*, recovery of the polypeptide from the whole fermentation broth, or from the cell-free fermentation broth, by polypeptide crystal harvest, by filtration, e.g. depth filtration (by use of filter aids or packed filter medias, cloth filtration in chamber filters, rotary-drum filtration, drum filtration, rotary vacuum-drum filters, candle filters, horizontal leaf filters or similar, using sheed or pad filtration in framed or modular setups) or membrane filtration (using sheet filtration, module filtration, candle filtration, microfiltration, ultrafiltration in either cross flow, dynamic cross flow or dead end operation), or by centrifugation (using decanter centrifuges, disc stack centrifuges, hyrdo cyclones or similar), or by precipitating the polypeptide and using relevant solid-liquid separation methods to harvest the polypeptide from the broth media by use of classification separation by particle sizes. Recovery encompasses isolation and/or purification of the polypeptide.

**Sequence identity:** The relatedness between two amino acid sequences or between two nucleotide sequences is described by the parameter "sequence identity".

For purposes of the present invention, the sequence identity between two amino acid sequences is determined as the output of "longest identity" using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 6.6.0 or later. The parameters used are a gap open penalty of 10, a gap extension penalty of 0.5, and the EBLOSUM62 (EMBOSS version of BLOSUM62) substitution matrix. In order for the Needle program to report the longest identity, the -nobrief option must be specified in the command line. The output of Needle labeled "longest identity" is calculated as follows:

$$(\text{Identical Residues} \times 100)/(\text{Length of Alignment} - \text{Total Number of Gaps in Alignment})$$

For purposes of the present invention, the sequence identity between two polynucleotide sequences is determined as the output of "longest identity" using the Needleman-Wunsch algorithm (Needleman and Wunsch, 1970, supra) as implemented in the Needle program of the EMBOSS package (EMBOSS: The European Molecular Biology Open Software Suite, Rice et al., 2000, supra), preferably version 6.6.0 or later. The parameters used are a gap open penalty of 10, a gap extension penalty of 0.5, and the EDNAFULL (EMBOSS version of NCBI NUC4.4) substitution matrix. In order for the Needle program to report the longest identity, the nobrief option must be specified in the command line. The output of Needle labeled "longest identity" is calculated as follows:

$$(\text{Identical Nucleotides} \times 100)/(\text{Length of Alignment} - \text{Total Number of Gaps in Alignment})$$

**Signal Peptide:** A "signal peptide" is a sequence of amino acids attached to the N-terminal portion of a protein, which facilitates the secretion of the protein outside the cell. The mature form of an extracellular protein lacks the signal peptide, which is cleaved off during the secretion process.

**Subsequence:** The term "subsequence" means a polynucleotide having one or more nucleotides absent from the 5' and/or 3' end of a mature polypeptide coding sequence; wherein the subsequence encodes a fragment having deamidase activity.

**Thermal unfolding temperature:** The term "thermal unfolding temperature", also referred to as "melting temperature", "$T_m$" or "midpoint unfolding temperature", means a temperature where approximately 50% of the fusion protein is unfolded. Typically, the fraction of folded protein is dominant (>99.999%) at room temperature and decreases as the temperature approaches the melting temperature, $T_m$. At $T_m$, ca. 50% of the molecules are in the folded state, and ca. 50% are in the unfolded state. At temperatures above $T_m$, the unfolded state becomes the dominant species (>50%). A preferred method to determine the termal unfolding temperature of the fusion proteins of the invention is nanoDSF. Using nanoDSF, determination of $T_m$ can be carried out at 330 nm, 350 nm, and/or with a ratio of 330nm/350nm, wherein $T_m$ equals the temperature of the inflection point (IP) of the first derivative. At the inflection point, the first derivative reaches a local maximum or minimum, and the second derivate has an isolated zero. For the present invention, $T_m$ or thermal unfolding temperature is the temperature of the inflection point (IP) of the first derivative determined by nanoDSF at 330 nm as described in Example 2.

Other methods for determining the thermal unfolding temperature are a thermal shift assay as described in Current Protocols in Protein Science: "Analysis of protein stability and ligand interactions by thermal shift assay" (K. Huynh and C.L. Partch, 2015) or the methods described in Encyclopedia of Industrial Biotechnology: "Proteins: Thermal Unfolding" (R. Lonescu and L. Shi, 2009).

Thermal unfolding represents an important tool to evaluate protein stability. In order to assess the protein preference to maintain its folded (active) conformation, the protein is exposed to increasing levels of denaturing stress (temperature) and protein stability is determined based on the stress level required to produce a significant fraction of unfolded protein. Factors that affect the thermal stability include, but are not limited to, protein structure, presence of ligands or excipients, binding strength between inhibitor and enzyme, and solvent conditions.

**Variant:** The term "variant" means a polypeptide having deamidase activity comprising a man-made mutation, i.e., a substitution, insertion (including extension), and/or deletion (e.g., truncation), at one or more positions. A substitution means replacement of the amino acid occupying a position with a different amino acid; a deletion means removal of the amino acid occupying a position; and an insertion means adding 1-5 amino acids (e.g., 1-3 amino acids, in particular, 1 amino acid) adjacent to and immediately following the amino acid occupying a position.

**Wild-type:** The term "wild-type" in reference to an amino acid sequence or nucleic acid sequence means that the amino acid sequence or nucleic acid sequence is a native or naturally-occurring sequence. As used herein, the term "naturally-occurring" refers to anything (e.g., proteins, amino acids, or nucleic acid sequences) that is found in nature. Conversely, the term "non-naturally occurring" refers to anything that is not found in nature (e.g., recombinant nucleic acids and protein sequences produced in the laboratory or modification of the wild-type sequence).

**Conventions for Designation of Variants:**

For purposes of the present invention, the polypeptide disclosed in SEQ ID NO: 5 is used to determine the corresponding amino acid positions in another propeptide and/or deamidase. The amino acid sequence of another propeptide and/or deamidase is aligned with the polypeptide disclosed in SEQ ID NO: 5, and based on the alignment, the amino acid position number corresponding to any amino acid residue in the polypeptide disclosed in SEQ ID NO: 5 is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix.

In describing the variants of the present invention, the nomenclature described below is adapted for ease of reference. The accepted IUPAC single letter or three letter amino acid abbreviation is employed.

Substitutions. For an amino acid substitution, the following nomenclature is used: Original amino acid, position, substituted amino acid. Accordingly, the substitution of threonine at position 226 with alanine is designated as "Thr226Ala" or "T226A". Multiple mutations are separated by addition marks ("+"), *e.g.*, "Gly205Arg + Ser411Phe" or "G205R + S411F", representing substitutions at positions 205 and 411 of glycine (G) with arginine (R) and serine (S) with phenylalanine (F), respectively.

Deletions. For an amino acid deletion, the following nomenclature is used: Original amino acid, position, *. Accordingly, the deletion of glycine at position 195 is designated as "Gly195*" or "G195*". Multiple deletions are separated by addition marks ("+"), *e.g.*, "Gly195* + Ser411*" or "G195* + S411*".

Insertions. For an amino acid insertion, the following nomenclature is used: Original amino acid, position, original amino acid, inserted amino acid. Accordingly, the insertion of lysine after glycine at position 195 is designated "Gly195GlyLys" or "G195GK". An insertion of multiple amino acids is designated [Original amino acid, position, original amino acid, inserted amino acid #1, inserted amino acid #2; etc.]. For example, the insertion of lysine and alanine after glycine at position 195 is indicated as "Gly195GlyLysAla" or "G195GKA".

In such cases, the inserted amino acid residue(s) are numbered by the addition of lower case letters to the position number of the amino acid residue preceding the inserted amino acid residue(s). In the above example, the sequence would thus be:

| Parent | Variant |
|--------|---------|
| 195 | 195 195a 195b |
| G | G - K - A |

Multiple alterations. Variants comprising multiple alterations are separated by addition marks ("+"), e.g., "Arg170Tyr+Gly195Glu" or "R170Y+G195E" representing a substitution of arginine and glycine at positions 170 and 195 with tyrosine and glutamic acid, respectively.

Different alterations. Where different alterations can be introduced at a position, the different alterations are separated by a comma, e.g., "Arg170Tyr,Glu" represents a substitution of arginine at position 170 with tyrosine or glutamic acid. Thus, "Tyr167Gly,Ala + Arg170Gly,Ala" designates the following variants:

"Tyr167Gly+Arg170Gly", "Tyr167Gly+Arg170Ala", "Tyr167Ala+Arg170Gly", and
"Tyr167Ala+Arg 170Ala".

## DETAILED DESCRIPTION

### Recombinant Deamidase Fusion Polypeptides

The present invention relates to recombinant deamidase fusion polypeptides (pro-form). In an aspect, the invention relates to recombinant fusion polypeptides comprising or consisting of

(a) a first polypeptide comprising a deamidase inhibitory domain, and
(b) a second polypeptide having deamidase activity;

wherein the fusion polypeptide has a thermal unfolding temperature in the range of 65-82°C; and the fusion polypeptide has less than 50% deamidase activity relative to the deamidase activity of the second polypeptide.

In an embodiment, the thermal unfolding temperature is in the range of 65-80°C, preferably 67-80°C, and more preferably 70-80°C. The thermal unfolding temperature may be measured as nanoDSF thermal unfolding temperature, for example as described in Example 2.

In an embodiment, the C-terminal end of the first polypeptide is located before the N-terminal end of the second polypeptide.

In an embodiment, the first polypeptide is located in proximity of the N-terminal end of the fusion polyeptide; preferably within 30 amino acids, such as within 20 amino acids or within 10 amino acids, from the N-terminal end of the fusion polyeptide. Likewise, the second polypeptide may be located in proximity of the C-terminal end of the fusion polyeptide; preferably within 30 amino acids, such as within 20 amino acids or within 10 amino acids, from the C-terminal end of the fusion polyeptide.

In an embodiment, the fusion polypeptide has less than 40% deamidase activity, preferably less than 35% deamidase activity, as compared to the second polypeptide (leakage activity).

In an embodiment, the first polypeptide comprises the amino acid sequence motif of SEQ ID NO: 24 and/or the amino acid sequence motif of SEQ ID NO: 25.

In an embodiment, the second polypeptide comprises an amino acid sequence motif selected from the group consisting of SEQ ID NO: 21, SEQ ID NO: 22, SEQ ID NO: 23, and combinations thereof.

In an embodiment, the first polypeptide comprises an amino acid change in a position corresponding to a position selected from the group consisting of 23, 38, 45, 94, and 99, of SEQ ID NO: 2. Preferably, the first polypeptide comprises an amino acid change in a position corresponding to a position selected from the group consisting of V23, F38, Y45, Y94, and F99, of SEQ ID NO: 2. More preferably, the first polypeptide comprises an amino acid change in a position corresponding to a position selected from the group consisting of V23S, F38C, Y45G, Y45T, Y94P, F99A, F99G, and F99K, of SEQ ID NO: 2.

In an embodiment, the fusion polypeptide has at least 60%, at least 70%, at least 80%, at least 90%, at least 95%, at least 96%, at least 97%, at least 98%, or at least 99%, but less than 100%, amino acid sequence identity to an amino acid sequence selected from the group consisting of SEQ ID NO: 5, SEQ ID NO: 10, SEQ ID NO: 15, and SEQ ID NO: 20.

In an embodiment, the fusion polypeptide has 1-30 alterations (e.g., substitutions, deletions and/or insertions), preferably 1-20 alterations, 1-10 alterations, or 1-5 alterations, in particular substitutions, as compared to an amino acid sequence selected from the group consisting of SEQ ID NO: 5, SEQ ID NO: 10, SEQ ID NO: 15, and SEQ ID NO: 20.

In an embodiment, the first polypeptide has at least 60%, at least 70%, at least 80%, at least 90%, at least 95%, at least 96%, at least 97%, at least 98%, or at least 99%, but less than 100%, amino acid sequence identity to an amino acid sequence selected from the group consisting of SEQ ID NO: 2, SEQ ID NO: 7, SEQ ID NO: 12, and SEQ ID NO: 17.

In an embodiment, the second polypeptide has 1-30 alterations (e.g., substitutions, deletions and/or insertions), preferably 1-20 alterations, 1-10 alterations, or 1-5 alterations, in particular substitutions, as compared to an amino acid sequence selected from the group consisting of SEQ ID NO: 4, SEQ ID NO: 9, SEQ ID NO: 14, and SEQ ID NO: 19.

In an embodiment, the second polypeptide has at least 60%, at least 70%, at least 80%, at least 90%, at least 95%, at least 96%, at least 97%, at least 98%, or at least 99% amino acid sequence identity to an amino acid sequence selected from the group consisting of SEQ ID NO: 4, SEQ ID NO: 9, SEQ ID NO: 14, and SEQ ID NO: 19.

In an embodiment, the second polypeptide has up to 30 alterations (e.g., substitutions, deletions and/or insertions), preferably up to 20 alterations, up to 10 alterations, or up to 5 alterations, in particular substitutions, as compared to an amino acid sequence selected from the group consisting of SEQ ID NO: 4, SEQ ID NO: 9, SEQ ID NO: 14, and SEQ ID NO: 19.

In an embodiment, the first polypeptide comprises a cleavage site for a site-specific endopeptidase, such as glutamyl endopeptidase or trypsin. Preferably, the cleavage site is located within 20 amino acids, 15 amino acids, or 10 amino acids of the C-terminal end.

In another aspect, the invention provides a method for producing a polypeptide having deamidase activity, comprising contacting the fusion polypeptide of the invention with a site-specific endopeptidase, such as glutamyl endopeptidase, trypsin-like endopeptidases, or chymotrypsin-like endopeptidases, to separate the first polypeptide from the second polypeptide.

In yet another aspect, the invention provides a composition exhibiting deamidase activity, comprising the first polypeptide and the second polypeptide of the fusion polypeptide, wherein the first and second polypeptides are not covalently linked. Preferably the composition further comprises a site-specific endopeptidase.

In an embodiment, the composition is a liquid composition comprising

(a) 0.001-25% w/w of the first polypeptide,
(b) 0.001-25% w/w of the second polypeptide,
(c) polyol(s), and
(d) water.

In yet another aspect, the invention provides a method for modifying a protein, comprising contacting the protein with the composition exhibiting deamidase activity of the invention. Preferably, the modification is a deamidation of a glutamine residue (conversion of glutamine to glutamate).

Amino acid alterations, as described above, may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding module.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant molecules are tested for deamidase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide, and/or be inferred from sequence homology and conserved catalytic machinery with a related polypeptide or within a polypeptide or protein family with polypeptides/proteins descending from a common ancestor, typically having similar three-dimensional structures, functions, and significant sequence similarity. Additionally or alternatively, protein structure prediction tools can be used for protein structure modelling to identify essential amino acids and/or active sites of polypeptides. See, for example, Jumper et al., 2021, "Highly accurate protein structure prediction with AlphaFold", Nature 596: 583-589.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; US 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

In an aspect, the polypeptide is isolated.

In another aspect, the polypeptide is purified.

In another aspect, the invention provides a method for producing a polypeptide having deamidase activity, comprising contacting the fusion polypeptide of the invention with an endopeptidase to separate the first polypeptide from the second polypeptide.

In yet another aspect, the invention provides a composition exhibiting deamidase activity, comprising the separated first polypeptide and second polypeptide of the fusion polypeptide of the invention.

**Sources of Wildtype Deamidase Fusion Polypeptides**

Wildtype deamidase fusion polypeptides (pro-forms), which can be used to make the recombinant fusion polypeptides of the present invention may be obtained from microorganisms (donor strains) of any genus. The wildtype fusion polypeptides may subsequently be modified in the propeptide (first polypeptide) to reduce the thermal unfolding temperature of the fusion polypeptide in accordance with the invention. Several examples of wildtype fusion polypeptides obtained from *Chryseobacterium* species are shown in Example 5. For purposes of the present invention, the term "obtained from" as used herein in connection with a given source shall mean that the polypeptide encoded by a polynucleotide is produced by the source or by a strain in which the polynucleotide of the invention has been inserted. In one aspect, the polypeptide obtained from a given source is secreted extracellularly.

In an embodiment, the wildtype fusion polypeptide is obtained from a *Chryseobacterium* species.

The wildtype fusion polypeptides may be identified and obtained from sources including microorganisms isolated from nature (e.g., soil, composts, water, etc.) or DNA samples obtained directly from natural materials (e.g., soil, composts, water, etc.). Techniques for isolating microorganisms and DNA directly from natural habitats are well known in the art. A polynucleotide encoding the polypeptide may then be obtained by similarly screening a genomic DNA or cDNA library of another microorganism or mixed DNA sample. Once a polynucleotide encoding a polypeptide has been detected with the probe(s), the polynucleotide can be isolated or cloned by utilizing techniques that are known to those of ordinary skill in the art (see, e.g., Davis et al., 2012, Basic Methods in Molecular Biology, Elsevier).

**Polynucleotides**

The present invention also relates to polynucleotides encoding a polypeptide of the present invention, as described herein.

The polynucleotide may be mutated by introduction of nucleotide substitutions that do not result in a change in the amino acid sequence of the polypeptide, but which correspond to the codon usage of the host organism intended for production of the enzyme, or by introduction of nucleotide substitutions that may give rise to a different amino acid sequence. For a general description of nucleotide substitution, see, e.g., Ford et al., 1991, Protein Expression and Purification 2: 95-107.

In an aspect, the polynucleotide is isolated.

In another aspect, the polynucleotide is purified.

**Nucleic Acid Constructs**

The present invention also relates to nucleic acid constructs comprising a polynucleotide of the present invention, wherein the polynucleotide is operably linked to one or more control sequences that direct the expression of the coding sequence in a suitable host cell under conditions compatible with the control sequences.

The polynucleotide may be manipulated in a variety of ways to provide for expression of the polypeptide. Manipulation of the polynucleotide prior to its insertion into a vector may be desirable or necessary depending on the expression vector. Techniques for modifying polynucleotides utilizing recombinant DNA methods are well known in the art.

Promoters

The control sequence may be a promoter, a polynucleotide that is recognized by a host cell for expression of a polynucleotide encoding a polypeptide of the present invention. The promoter contains transcriptional control sequences that mediate the expression of the polypeptide. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the polynucleotide of the present invention in a bacterial host cell are described in Sambrook et al., 1989, Molecular Cloning: A Laboratory Manual, Cold Spring Harbor Lab., NY, Davis et al., 2012, supra, and Song et al., 2016, PLOS One 11(7): e0158447.

Examples of suitable promoters for directing transcription of the polynucleotide of the present invention in a filamentous fungal host cell are promoters obtained from Aspergillus, Fusarium, Rhizomucor and Trichoderma cells, such as the promoters described in Mukherjee et al., 2013, "Trichoderma: Biology and Applications", and by Schmoll and Dattenböck, 2016, "Gene Expression Systems in Fungi: Advancements and Applications", Fungal Biology.

For expression in a yeast host, examples of useful promoters are described by Smolke et al., 2018, "Synthetic Biology: Parts, Devices and Applications" (Chapter 6: Constitutive and Regulated Promoters in Yeast: How to Design and Make Use of Promoters in S. cerevisiae), and by Schmoll and Dattenböck, 2016, "Gene Expression Systems in Fungi: Advancements and Applications", Fungal Biology.

Terminators

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for bacterial host cells may be obtained from the genes for Bacillus clausii alkaline protease (aprH), Bacillus licheniformis alpha-amylase (amyL), and Escherichia coli ribosomal RNA (rrnB).

Preferred terminators for filamentous fungal host cells may be obtained from Aspergillus or Trichoderma species, such as obtained from the genes for Aspergillus niger glucoamylase, Trichoderma reesei beta-glucosidase, Trichoderma reesei cellobiohydrolase I, and Trichoderma reesei endoglucanase I, such as the terminators described in Mukherjee et al., 2013, "Trichoderma: Biology and Applications", and by Schmoll and Dattenböck, 2016, "Gene Expression Systems in Fungi: Advancements and Applications", Fungal Biology.

Preferred terminators for yeast host cells may be obtained from the genes for Saccharomyces cerevisiae enolase, Saccharomyces cerevisiae cytochrome C (CYC1), and Saccharomyces cerevisiae glyceraldehyde-3-phosphate dehydrogenase. Other useful terminators for yeast host cells are described by Romanos et al., 1992, Yeast 8: 423-488.

mRNA Stabilizers

The control sequence may also be an mRNA stabilizer region downstream of a promoter and upstream of the coding sequence of a gene which increases expression of the gene.

Examples of suitable mRNA stabilizer regions are obtained from a Bacillus thuringiensis cryIIIA gene (WO 94/25612) and a Bacillus subtilis SP82 gene (Hue et al., 1995, J. Bacteriol. 177: 3465-3471).

Examples of mRNA stabilizer regions for fungal cells are described in Geisberg et al., 2014, Cell 156(4): 812-824, and in Morozov et al., 2006, Eukaryotic Cell 5(11): 1838-1846.

Leader Sequences

The control sequence may also be a leader, a non-translated region of an mRNA that is important for translation by the host cell. The leader is operably linked to the 5'-terminus of the polynucleotide encoding the polypeptide. Any leader that is functional in the host cell may be used.

Suitable leaders for bacterial host cells are described by Hambraeus et al., 2000, Microbiology 146(12): 3051-3059, and by Kaberdin and Bläsi, 2006, FEMS Microbiol. Rev. 30(6): 967-979.

Preferred leaders for filamentous fungal host cells may be obtained from the genes for Aspergillus oryzae TAKA amylase and Aspergillus nidulans triose phosphate isomerase.

Suitable leaders for yeast host cells may be obtained from the genes for Saccharomyces cerevisiae enolase (ENO-1), Saccharomyces cerevisiae 3-phosphoglycerate kinase, Saccharomyces cerevisiae alpha-factor, and Saccharomyces cerevisiae alcohol dehydrogenase/glyceraldehyde-3-phosphate dehydrogenase (ADH2/GAP).

Polyadenylation Sequences

The control sequence may also be a polyadenylation sequence, a sequence operably linked to the 3'-terminus of the polynucleotide which, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for Aspergillus nidulans anthranilate synthase, Aspergillus niger glucoamylase, Aspergillus niger alpha-glucosidase, Aspergillus oryzae TAKA amylase, and Fusarium oxysporum trypsin-like protease.

Useful polyadenylation sequences for yeast host cells are described by Guo and Sherman, 1995, Mol. Cellular Biol. 15: 5983-5990.

Signal Peptides

The control sequence may also be a signal peptide coding region that encodes a signal peptide linked to the N-terminus of a polypeptide and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is heterologous to the coding sequence. A heterologous signal peptide coding sequence may be required where the coding sequence does not naturally contain a signal peptide coding sequence. Alternatively, a heterologous signal peptide coding sequence may simply replace the natural signal peptide coding sequence to enhance secretion of the polypeptide. Any signal peptide coding sequence that directs the expressed polypeptide into the secretory pathway of a host cell may be used.

Effective signal peptide coding sequences for bacterial host cells are the signal peptide coding sequences obtained from the genes for Bacillus NCIB 11837 maltogenic amylase, Bacillus licheniformis subtilisin, Bacillus licheniformis beta-lactamase, Bacillus stearothermophilus alpha-amylase, Bacillus stearothermophilus neutral proteases (nprT, nprS, nprM), and Bacillus subtilis prsA. Further signal peptides are described by Freudl, 2018, Microbial Cell Factories 17: 52.

Effective signal peptide coding sequences for filamentous fungal host cells are the signal peptide coding sequences obtained from the genes for Aspergillus niger neutral amylase, Aspergillus niger glucoamylase, Aspergillus oryzae TAKA amylase, Humicola insolens cellulase, Humicola insolens endoglucanase V, Humicola lanuginosa lipase, and Rhizomucor miehei aspartic proteinase, such as the signal peptide described by Xu et al., 2018, Biotechnology Letters 40: 949-955

Useful signal peptides for yeast host cells are obtained from the genes for Saccharomyces cerevisiae alpha-factor and Saccharomyces cerevisiae invertase. Other useful signal peptide coding sequences are described by Romanos et al., 1992, supra.

## Regulatory Sequences

It may also be desirable to add regulatory sequences that regulate expression of the polypeptide relative to the growth of the host cell. Examples of regulatory sequences are those that cause expression of the gene to be turned on or off in response to a chemical or physical stimulus, including the presence of a regulatory compound. Regulatory sequences in prokaryotic systems include the lac, tac, and trp operator systems. In yeast, the ADH2 system or GAL1 system may be used. In filamentous fungi, the Aspergillus niger glucoamylase promoter, Aspergillus oryzae TAKA alpha-amylase promoter, and Aspergillus oryzae glucoamylase promoter, Trichoderma reesei cellobiohydrolase I promoter, and Trichoderma reesei cellobiohydrolase II promoter may be used. Other examples of regulatory sequences are those that allow for gene amplification. In fungal systems, these regulatory sequences include the dihydrofolate reductase gene that is amplified in the presence of methotrexate, and the metallothionein genes that are amplified with heavy metals.

## Transcription Factors

The control sequence may also be a transcription factor, a polynucleotide encoding a polynucleotide-specific DNA-binding polypeptide that controls the rate of the transcription of genetic information from DNA to mRNA by binding to a specific polynucleotide sequence. The transcription factor may function alone and/or together with one or more other polypeptides or transcription factors in a complex by promoting or blocking the recruitment of RNA polymerase. Transcription factors are characterized by comprising at least one DNA-binding domain which often attaches to a specific DNA sequence adjacent to the genetic elements which are regulated by the transcription factor. The transcription factor may regulate the expression of a protein of interest either directly, i.e., by activating the transcription of the gene encoding the protein of interest by binding to its promoter, or indirectly, i.e., by activating the transcription of a further transcription factor which regulates the transcription of the gene encoding the protein of interest, such as by binding to the promoter of the further transcription factor. Suitable transcription factors for fungal host cells are described in WO 2017/144177. Suitable transcription factors for prokaryotic host cells are described in Seshasayee et al., 2011, Subcellular Biochemistry 52: 7-23, as well in Balleza et al., 2009, FEMS Microbiol. Rev. 33(1): 133-151.

## Expression Vectors

The present invention also relates to recombinant expression vectors comprising a polynucleotide of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the polypeptide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, i.e., a vector that exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

The vector preferably contains at least one element that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous recombination, such as homology-directed repair (HDR), or non-homologous recombination, such as non-homologous end-joining (NHEJ).

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate in vivo.

More than one copy of a polynucleotide of the present invention may be inserted into a host cell to increase production of a polypeptide. For example, 2 or 3 or 4 or 5 or more copies are inserted into a host cell. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

**Host Cells**

The present invention also relates to recombinant host cells, comprising a polynucleotide of the present invention operably linked to one or more control sequences that direct the production of a polypeptide of the present invention.

A construct or vector comprising a polynucleotide is introduced into a host cell so that the construct or vector is maintained as a chromosomal integrant or as a self-replicating extrachromosomal vector as described earlier. The choice of a host cell will to a large extent depend upon the gene encoding the polypeptide and its source. The polypeptide can be native or heterologous to the recombinant host cell. Also, at least one of the one or more control sequences can be heterologous to the polynucleotide encoding the polypeptide. The recombinant host cell may comprise a single copy, or at least two copies, e.g., three, four, five, or more copies of the polynucleotide of the present invention.

The host cell may be any microbial cell useful in the recombinant production of a polypeptide of the present invention, e.g., a prokaryotic cell or a fungal cell.

The prokaryotic host cell may be any Gram-positive or Gram-negative bacterium. Gram-positive bacteria include, but are not limited to, Bacillus, Clostridium, Enterococcus, Geobacillus, Lactobacillus, Lactococcus, Oceanobacillus, Staphylococcus, Streptococcus, and Streptomyces. Gram-negative bacteria include, but are not limited to, Campylobacter, E. coli, Flavobacterium, *Fusobacterium*, Helicobacter, *Ilyobacter*, *Neisseria*, Pseudomonas, Salmonella, and *Ureaplasma.*

The bacterial host cell may be any Bacillus cell including, but not limited to, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, and Bacillus thuringiensis cells. In an embodiment, the Bacillus cell is a Bacillus amyloliquefaciens, Bacillus licheniformis and Bacillus subtilis cell.

For purposes of this invention, Bacillus classes/genera/species shall be defined as described in Patel and Gupta, 2020, Int. J. Syst. Evol. Microbiol. 70: 406-438.

The bacterial host cell may also be any Streptococcus cell including, but not limited to, Streptococcus equisimilis, Streptococcus pyogenes, Streptococcus uberis, and Streptococcus equi subsp. Zooepidemicus cells.

The bacterial host cell may also be any Streptomyces cell including, but not limited to, Streptomyces achromogenes, Streptomyces avermitilis, Streptomyces coelicolor, Streptomyces griseus, and Streptomyces lividans cells.

Methods for introducing DNA into prokaryotic host cells are well-known in the art, and any suitable method can be used including but not limited to protoplast transformation, competent cell transformation, electroporation, conjugation, transduction, with DNA introduced as linearized or as circular polynucleotide. Persons skilled in the art will be readily able to identify a suitable method for introducing DNA into a given prokaryotic cell depending, e.g., on the genus. Methods for introducing DNA into prokaryotic host cells are for example described in Heinze et al., 2018, BMC Microbiology 18:56, Burke et al., 2001, Proc. Natl. Acad. Sci. USA 98: 6289-6294, Choi et al., 2006, J. Microbiol. Methods 64: 391-397, and Donald et al., 2013, J. Bacteriol. 195(11): 2612-2620.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

Fungal cells may be transformed by a process involving protoplast-mediated transformation, Agrobacterium-mediated transformation, electroporation, biolistic method and shock-wave-mediated transformation as reviewed by Li et al., 2017, Microbial Cell Factories 16: 168 and procedures described in EP 238023, Yelton et al., 1984, Proc. Natl. Acad. Sci. USA 81: 1470-1474, Christensen et al., 1988, Bio/Technology 6: 1419-1422, and Lubertozzi and Keasling, 2009, Biotechn. Advances 27: 53-75. However, any method known in the art for introducing DNA into a fungal host cell can be used, and the DNA can be introduced as linearized or as circular polynucleotide.

The fungal host cell may be a yeast cell. "Yeast" as used herein includes ascosporogenous yeast (Endomycetales), basidiosporogenous yeast, and yeast belonging to the Fungi Imperfecti (Blastomycetes). For purposes of this invention, yeast shall be defined as described in Biology and Activities of Yeast (Skinner, Passmore, and Davenport, editors, Soc. App. Bacteriol. Symposium Series No. 9, 1980).

The yeast host cell may be a Candida, Hansenula, Kluyveromyces, Pichia, Saccharomyces, Schizosaccharomyces, or Yarrowia cell, such as a Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis, or Yarrowia lipolytica cell. In a preferred embodiment, the yeast host cell is a Pichia or Komagataella cell, e.g., a Pichia pastoris cell (Komagataella phaffii).

The fungal host cell may be a filamentous fungal cell. "Filamentous fungi" include all filamentous forms of the subdivision Eumycota and Oomycota (as defined by Hawksworth et al., 1995, supra). The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as Saccharomyces cerevisiae is by budding of a unicellular thallus and carbon catabolism may be fermentative.

The filamentous fungal host cell may be an Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes, or Trichoderma cell. In a preferred embodiment, the filamentous fungal host cell is an Aspergillus, Trichoderma or Fusarium cell. In a further preferred embodiment, the filamentous fungal host cell is an Aspergillus niger, Aspergillus oryzae, Trichoderma reesei, or Fusarium venenatum cell.

For example, the filamentous fungal host cell may be an Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, *Chrysosporium lucknowense*, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Talaromyces emersonii, Thielavia terrestris, Trametes villosa, Trametes versicolor, Trichoderma harzianum, Trichoderma koningii, Trichoderma longibrachiatum, Trichoderma reesei, or Trichoderma viride cell.

In an aspect, the host cell is isolated.

In another aspect, the host cell is purified.

## Methods of Production

The present invention also relates to methods of producing a polypeptide of the present invention, comprising (a) cultivating a recombinant host cell of the present invention under conditions conducive for production of the polypeptide; and optionally, (b) recovering the polypeptide.

The host cell is cultivated in a nutrient medium suitable for production of the polypeptide using methods known in the art. For example, the cell may be cultivated by shake flask cultivation, or small-scale or large-scale fermentation (including continuous, batch, fed-batch, or solid-state, and/or microcarrier-based fermentations) in laboratory or industrial fermentors in a suitable medium and under conditions allowing the polypeptide to be expressed and/or isolated. Suitable media are available from commercial suppliers or may be prepared according to published compositions (e.g., in catalogues of the American Type Culture Collection). If the polypeptide is secreted into the nutrient medium, the polypeptide can be recovered directly from the medium. If the polypeptide is not secreted, it can be recovered from cell lysates.

The polypeptide may be detected using methods known in the art that are specific for the polypeptide, including, but not limited to, the use of specific antibodies, formation of an enzyme product, disappearance of an enzyme substrate, or an assay determining the relative or specific activity of the polypeptide.

The polypeptide may be recovered from the medium using methods known in the art, including, but not limited to, collection, centrifugation, filtration, extraction, spray-drying, evaporation, or precipitation. In one aspect, a whole fermentation broth comprising the polypeptide is recovered. In another aspect, a cell-free fermentation broth comprising the polypeptide is recovered.

The polypeptide may be purified by a variety of procedures known in the art to obtain substantially pure polypeptides and/or polypeptide fragments (see, e.g., Wingfield, 2015, Current Protocols in Protein Science; 80(1): 6.1.1-6.1.35; Labrou, 2014, Protein Downstream Processing, 1129: 3-10).

In an alternative aspect, the polypeptide is not recovered.

**Deamidase Granules**

The present invention also relates to enzyme granules/particles comprising a polypeptide of the invention. In an embodiment, the granule comprises a core, and optionally one or more coatings (outer layers) surrounding the core.

The core may have a diameter, measured as equivalent spherical diameter (volume based average particle size), of 20-2000 μm, particularly 50-1500 μm, 100-1500 μm or 250-1200 μm. The core diameter, measured as equivalent spherical diameter, can be determined using laser diffraction, such as using a Malvern Mastersizer and/or the method described under ISO13320 (2020).

In an embodiment, the core comprises a polypeptide having deamidase activity of the present invention.

The core may include additional materials such as fillers, fiber materials (cellulose or synthetic fibers), stabilizing agents, solubilizing agents, suspension agents, viscosity regulating agents, light spheres, plasticizers, salts, lubricants and fragrances.

The core may include a binder, such as synthetic polymer, wax, fat, or carbohydrate.

The core may include a salt of a multivalent cation, a reducing agent, an antioxidant, a peroxide decomposing catalyst and/or an acidic buffer component, typically as a homogenous blend.

The core may include an inert particle with the polypeptide absorbed into it, or applied onto the surface, e.g., by fluid bed coating.

The core may have a diameter of 20-2000 μm, particularly 50-1500 μm, 100-1500 μm or 250-1200 μm.

The core may be surrounded by at least one coating, e.g., to improve the storage stability, to reduce dust formation during handling, or for coloring the granule. The optional coating(s) may include a salt coating, or other suitable coating materials, such as polyethylene glycol (PEG), methyl hydroxy-propyl cellulose (MHPC) and polyvinyl alcohol (PVA).

The coating may be applied in an amount of at least 0.1% by weight of the core, e.g., at least 0.5%, at least 1%, at least 5%, at least 10%, or at least 15%. The amount may be at most 100%, 70%, 50%, 40% or 30%.

The coating is preferably at least 0.1 μm thick, particularly at least 0.5 μm, at least 1 μm or at least 5 μm. In some embodiments, the thickness of the coating is below 100 μm, such as below 60 μm, or below 40 μm.

The coating should encapsulate the core unit by forming a substantially continuous layer. A substantially continuous layer is to be understood as a coating having few or no holes, so that the core unit has few or no uncoated areas. The layer or coating should, in particular, be homogeneous in thickness.

The coating can further contain other materials as known in the art, e.g., fillers, antisticking agents, pigments, dyes, plasticizers and/or binders, such as titanium dioxide, kaolin, calcium carbonate or talc.

A salt coating may comprise at least 60% by weight of a salt, e.g., at least 65%, at least 70%, at least 75%, at least 80%, at least 85%, at least 90%, at least 95% or at least 99% by weight.

To provide acceptable protection, the salt coating is preferably at least 0.1 μm thick, e.g., at least 0.5 μm, at least 1 μm, at least 2 μm, at least 4 μm, at least 5 μm, or at least 8 μm. In a particular embodiment, the thickness of the salt coating is below 100 μm, such as below 60 μm, or below 40 μm.

The salt may be added from a salt solution where the salt is completely dissolved or from a salt suspension wherein the fine particles are less than 50 μm, such as less than 10 μm or less than 5 μm.

The salt coating may comprise a single salt or a mixture of two or more salts. The salt may be water soluble, in particular, having a solubility at least 0.1 g in 100 g of water at 20°C, preferably at least 0.5 g per 100 g water, e.g., at least 1 g per 100 g water, e.g., at least 5 g per 100 g water.

The salt may be an inorganic salt, e.g., salts of sulfate, sulfite, phosphate, phosphonate, nitrate, chloride or carbonate or salts of simple organic acids (less than 10 carbon atoms, e.g., 6 or less carbon atoms) such as citrate, malonate or acetate. Examples of cations in these salts are alkali or earth alkali metal ions, the ammonium ion or metal ions of the first transition series, such as sodium, potassium, magnesium, calcium, zinc or aluminum. Examples of anions include chloride, bromide, iodide, sulfate, sulfite, bisulfite, thiosulfate, phosphate, monobasic phosphate, dibasic phosphate, hypophosphite, dihydrogen pyrophosphate, tetraborate, borate, carbonate, bicarbonate, metasilicate, citrate, malate, maleate, malonate, succinate, lactate, formate, acetate, butyrate, propionate, benzoate, tartrate, ascorbate or gluconate. In particular, alkali- or earth alkali metal salts of sulfate, sulfite, phosphate, phosphonate, nitrate, chloride or carbonate or salts of simple organic acids such as citrate, malonate or acetate may be used.

The salt in the coating may have a constant humidity at 20°C above 60%, particularly above 70%, above 80% or above 85%, or it may be another hydrate form of such a salt (e.g., anhydrate). The salt coating may be as described in WO 00/01793 or WO 2006/034710.

Specific examples of suitable salts are NaCl ($CH_{20°C}$=76%), $Na_2CO_3$ ($CH_{20°C}$=92%), $NaNO_3$ ($CH_{20°C}$=73%), $Na_2HPO_4$ ($CH_{20°C}$=95%), $Na_3PO_4$ ($CH_{25°C}$=92%), $NH_4Cl$ ($CH_{20°C}$ = 79.5%), $(NH_4)_2HPO_4$ ($CH_{20°C}$ = 93,0%), $NH_4H_2PO_4$ ($CH_{20°C}$ = 93.1%), $(NH_4)_2SO_4$ ($CH_{20°C}$=81.1%), KCl ($CH_{20°C}$=85%), $K_2HPO_4$ ($CH_{20°C}$=92%), $KH_2PO_4$ ($CH_{20°C}$=96.5%), $KNO_3$ ($CH_{20°C}$=93.5%), $Na_2SO_4$ ($CH_{20°C}$=93%), $K_2SO_4$ ($CH_{20°C}$=98%), $KHSO_4$ ($CH_{20°C}$=86%), $MgSO_4$ ($CH_{20°C}$=90%), $ZnSO_4$ ($CH_{20°C}$=90%) and sodium citrate ($CH_{25°C}$=86%). Other examples include $NaH_2PO_4$, $(NH_4)H_2PO_4$, $CuSO_4$, $Mg(NO_3)_2$ and magnesium acetate.

The salt may be in anhydrous form, or it may be a hydrated salt, i.e., a crystalline salt hydrate with bound water(s) of crystallization, such as described in WO 99/32595. Specific examples include anhydrous sodium sulfate ($Na_2SO_4$), anhydrous magnesium sulfate ($MgSO_4$), magnesium sulfate heptahydrate ($MgSO_4.7H_2O$), zinc sulfate heptahydrate ($ZnSO_4.7H_2O$), sodium phosphate dibasic heptahydrate ($Na_2HPO_4.7H_2O$), magnesium nitrate hexahydrate ($Mg(NO_3)_2(6H_2O)$), sodium citrate dihydrate and magnesium acetate tetrahydrate.

Preferably the salt is applied as a solution of the salt, e.g., using a fluid bed.

The coating materials can be waxy coating materials and film-forming coating materials. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591.

The granule may optionally have one or more additional coatings. Examples of suitable coating materials are polyethylene glycol (PEG), methyl hydroxy-propyl cellulose (MHPC) and polyvinyl alcohol (PVA). Examples of enzyme granules with multiple coatings are described in WO 93/07263 and WO 97/23606.

The core can be prepared by granulating a blend of the ingredients, e.g., by a method comprising granulation techniques such as crystallization, precipitation, pan-coating, fluid bed coating, fluid bed agglomeration, rotary atomization, extrusion, prilling, spheronization, size reduction methods, drum granulation, and/or high shear granulation.

Methods for preparing the core can be found in the Handbook of Powder Technology; Particle size enlargement by C. E. Capes; Vol. 1; 1980; Elsevier. Preparation methods include known feed and granule formulation technologies, e.g.,

(a) Spray dried products, wherein a liquid polypeptide-containing solution is atomized in a spray drying tower to form small droplets which during their way down the drying tower dry to form a polypeptide-containing particulate material. Very small particles can be produced this way (Michael S. Showell (editor); Powdered detergents; Surfactant Science Series; 1998; Vol. 71; pages 140-142; Marcel Dekker).

(b) Layered products, wherein the polypeptide is coated as a layer around a pre-formed inert core particle, wherein a polypeptide-containing solution is atomized, typically in a fluid bed apparatus wherein the pre-formed core particles are fluidized, and the polypeptide-containing solution adheres to the core particles and dries up to leave a layer of dry polypeptide on the surface of the core particle. Particles of a desired size can be obtained this way if a useful core particle of the desired size can be found. This type of product is described in, e.g., WO 97/23606.

(c) Absorbed core particles, wherein rather than coating the polypeptide as a layer around the core, the polypeptide is absorbed onto and/or into the surface of the core. Such a process is described in WO 97/39116.

(d) Extrusion or pelletized products, wherein a polypeptide-containing paste is pressed to pellets or under pressure is extruded through a small opening and cut into particles which are subsequently dried. Such particles usually have a considerable size because of the material in which the extrusion opening is made (usually a plate with bore holes) sets a limit on the allowable pressure drop over the extrusion opening. Also, very high extrusion pressures when using a small opening increase heat generation in the polypeptide paste, which is harmful to the polypeptide (Michael S. Showell (editor); Powdered detergents; Surfactant Science Series; 1998; Vol. 71; pages 140-142; Marcel Dekker).

(e) Prilled products, wherein a polypeptide-containing powder is suspended in molten wax and the suspension is sprayed, e.g., through a rotating disk atomizer, into a cooling chamber where the droplets quickly solidify (Michael S. Showell (editor); Powdered detergents; Surfactant Science Series; 1998; Vol. 71; pages 140-142; Marcel Dekker). The product obtained is one wherein the polypeptide is uniformly distributed throughout an inert material instead of being concentrated on its surface. US 4,016,040 and US 4,713,245 describe this technique.

(f) Mixer granulation products, wherein a polypeptide-containing liquid is added to a dry powder composition of conventional granulating components. The liquid and the powder in a suitable proportion are mixed and as the moisture of the liquid is absorbed in the dry powder, the components of the dry powder will start to adhere and agglomerate and particles will build up, forming granulates comprising the polypeptide. Such a process is described in US 4,106,991, EP 170360, EP 304332, EP 304331, WO 90/09440 and WO 90/09428. In a particular aspect of this process, various high-shear mixers can be used as granulators. Granulates consisting of polypeptide, fillers and binders etc. are mixed with cellulose fibers to reinforce the particles to produce a so-called T-granulate. Reinforced particles, are more robust, and release less enzymatic dust.

(g) Size reduction, wherein the cores are produced by milling or crushing of larger particles, pellets, tablets, briquettes etc. containing the polypeptide. The wanted core particle fraction is obtained by sieving the milled or crushed product. Over and undersized particles can be recycled. Size reduction is described in Martin Rhodes (editor); Principles of Powder Technology; 1990; Chapter 10; John Wiley & Sons.

(h) Fluid bed granulation. Fluid bed granulation involves suspending particulates in an air stream and spraying a liquid onto the fluidized particles via nozzles. Particles hit by spray droplets get wetted and become tacky. The tacky particles collide with other particles and adhere to them to form a granule.

(i) The cores may be subjected to drying, such as in a fluid bed drier. Other known methods for drying granules in the feed or enzyme industry can be used by the skilled person. The drying preferably takes place at a product temperature of from 25 to 90°C. For some polypeptides, it is important the cores comprising the polypeptide contain a low amount of water before coating with the salt. If water sensitive polypeptides are coated with a salt before excessive water is removed, the excessive water will be trapped within the core and may affect the activity of the polypeptide negatively. After drying, the cores preferably contain 0.1-10% w/w water.

Non-dusting granulates may be produced, e.g., as disclosed in US 4,106,991 and US 4,661,452 and may optionally be coated by methods known in the art.

The granulate may further comprise one or more additional enzymes, e.g., hydrolase, isomerase, ligase, lyase, oxidoreductase, and transferase. The one or more additional enzymes are preferably selected from the group consisting of acetylxylan esterase, acylglycerol lipase, amylase, alpha-amylase, beta-amylase, arabinofuranosidase, cellobiohydrolases, cellulase, feruloyl esterase, galactanase, alpha-galactosidase, beta-galactosidase, beta-glucanase, beta-glucosidase, lysophospholipase, lysozyme, alpha-mannosidase, beta-mannosidase (mannanase), phytase, phospholipase A1, phospholipase A2, phospholipase D, protease, pullulanase, pectin esterase, triacylglycerol lipase, xylanase, beta-xylosidase or any combination thereof. Each enzyme will then be present in more granules securing a more uniform distribution of the enzymes, and also reduces the physical segregation of different enzymes due to different particle sizes. Methods for producing multi-enzyme co-granulates is disclosed in the ip.com disclosure IPCOM000200739D.

Another example of formulation of polypeptides by the use of co-granulates is disclosed in WO 2013/188331.

The present invention also relates to protected polypeptides prepared according to the method disclosed in EP 238216.

**Liquid Formulations**

The present invention also relates to liquid compositions comprising a polypeptide of the invention. The composition may comprise an enzyme stabilizer (examples of which include polyols such as propylene glycol or glycerol, sugar or sugar alcohol, lactic acid, reversible protease inhibitor, boric acid, or a boric acid derivative, e.g., an aromatic borate ester, or a phenyl boronic acid derivative such as 4-formylphenyl boronic acid).

In some embodiments, filler(s) or carrier material(s) are included to increase the volume of such compositions. Suitable filler or carrier materials include, but are not limited to, various salts of sulfate, carbonate and silicate as well as talc, clay and the like. Suitable filler or carrier materials for liquid compositions include, but are not limited to, water or low molecular weight primary and secondary alcohols including polyols and diols. Examples of such alcohols include, but are not limited to, methanol, ethanol, propanol and isopropanol. In some embodiments, the compositions contain from about 5% to about 90% of such materials.

In an aspect, the liquid formulation comprises 20-80% w/w of polyol. In one embodiment, the liquid formulation comprises 0.001-2% w/w preservative.

In another embodiment, the invention relates to liquid formulations comprising:

(a) 0.001-25% w/w of a polypeptide having deamidase activity of the present invention;
(b) 20-80% w/w of polyol;
(c) optionally 0.001-2% w/w preservative; and
(d) water.

In another embodiment, the invention relates to liquid formulations comprising:

(a) 0.001-25% w/w of a polypeptide having deamidase activity of the present invention;
(b) 0.001-2% w/w preservative;
(c) optionally 20-80% w/w of polyol; and
(d) water.

In another embodiment, the liquid formulation comprises one or more formulating agents, such as a formulating agent selected from the group consisting of polyol, sodium chloride, sodium benzoate, potassium sorbate, sodium sulfate, potassium sulfate, magnesium sulfate, sodium thiosulfate, calcium carbonate, sodium citrate, dextrin, glucose, sucrose, sorbitol, lactose, starch, PVA, acetate and phosphate, preferably selected from the group consisting of sodium sulfate, dextrin, cellulose, sodium thiosulfate, kaolin and calcium carbonate. In one embodiment, the polyols is selected from the group consisting of glycerol, sorbitol, propylene glycol (MPG), ethylene glycol, diethylene glycol, triethylene glycol, 1,2-propylene glycol or 1,3-propylene glycol, dipropylene glycol, polyethylene glycol (PEG) having an average molecular weight below about 600 and polypropylene glycol (PPG) having an average molecular weight below about 600, more preferably selected from the group consisting of glycerol, sorbitol and propylene glycol (MPG) or any combination thereof.
In another embodiment, the liquid formulation comprises 20-80% polyol (i.e., total amount of polyol), e.g., 25-75% polyol, 30-70% polyol, 35-65% polyol, or 40-60% polyol. In one embodiment, the liquid formulation comprises 20-80% polyol, e.g., 25-75% polyol, 30-70% polyol, 35-65% polyol, or 40-60% polyol, wherein the polyol is selected from the group consisting of glycerol, sorbitol, propylene glycol (MPG), ethylene glycol, diethylene glycol, triethylene glycol, 1,2-propylene glycol or 1,3-propylene glycol, dipropylene glycol, polyethylene glycol (PEG) having an average molecular weight below about 600 and polypropylene glycol (PPG) having an average molecular weight below about 600. In one embodiment, the liquid formulation comprises 20-80% polyol (i.e., total amount of polyol), e.g., 25-75% polyol, 30-70% polyol, 35-65% polyol, or 40-60% polyol, wherein the polyol is selected from the group consisting of glycerol, sorbitol and propylene glycol (MPG).
In another embodiment, the preservative is selected from the group consisting of sodium sorbate, potassium sorbate, sodium benzoate and potassium benzoate or any combination thereof. In one embodiment, the liquid formulation comprises 0.02-1.5% w/w preservative, e.g., 0.05-1% w/w preservative or 0.1-0.5% w/w preservative. In one embodiment, the liquid formulation comprises 0.001-2% w/w preservative (i.e., total amount of preservative), e.g., 0.02-1.5% w/w preservative, 0.05-1% w/w preservative, or 0.1-0.5% w/w preservative, wherein the preservative is selected from the group consisting of sodium sorbate, potassium sorbate, sodium benzoate and potassium benzoate or any combination thereof.
In another embodiment, the liquid formulation further comprises one or more additional enzymes, e.g., hydrolase, isomerase, ligase, lyase, oxidoreductase, and transferase. The one or more additional enzymes are preferably selected from the group consisting of acetylxylan esterase, acylglycerol lipase, amylase, alpha-amylase, beta-amylase, arabinofuranosidase, cellobiohydrolases, cellulase, feruloyl esterase, galactanase, alpha-galactosidase, beta-galactosidase, beta-glucanase, beta-glucosidase, lysophospholipase, lysozyme, alpha-mannosidase, beta-mannosidase (mannanase), phytase, phospholipase A1, phospholipase A2, phospholipase D, protease, pullulanase, pectin esterase, triacylglycerol lipase, xylanase, beta-xylosidase or any combination thereof.

**Uses**

Protein deamidase can be applied on almost all types of proteins (plant proteins, animal protein, fermented proteins etc) where the enzyme will lower the isoelectric point of the proteins, and will, when the proteins are applied at a pH above the isoelectric point, improve solubility, electrostatic repulsion, improve different types of functionalities like foaming, emulsification, water binding etc., change affinity to flavours and off-flavours, gelling properties, improve thermostability etc. The enzymatically modified proteins can be applied as ingredients in various foods and beverages or the protein deamidade can be applied directly into a food production process like in a yogurt fermentation.
Plant proteins often have a low solubility and low functional properties. Deamidation is known to improve the solubility of plants proteins and partly of consequence hereof improve the functional properties including foaming activity, foaming stability, emulsification activity and emulsification stability. This has been observed on cross of several plant protein substrates including cereals protein like oat, wheat, corn protein and legume proteins like soy and pea protein, coconut protein etc. Negative attributes associated with the partly insoluble proteins like sandiness and grittiness are being mitigated by the enzymatic deamidation.

Examplewise enzymatically partly deamidated oat protein becomes essential fully soluble at neutral pH also leading to significantly improved emulsification properties. (Z-I Jiang at al, J Cereal Science (2015): 64: 126-132). One practical implication is the use of protein deamidase in the process for oat milk production, leading to an oat milk with increased protein content, improved foaming properties, a with a good stable emulsion being well suited to meet the requirement for the barista segment (WO 2014/123466). Similarly, emulsification and foaming properties are improved when soy protein isolate is enzymatically deamidated (I Suppavorasatit et al J. Agric. Food Chem (2011) 59: 11621-11628). For enzymatically deamidated pea protein isolate improved solubility, homogeneity, dispersibility, and suspendability and reduced beany flavour, grittiness and lumpiness have been observed (L Fang et al J, Agric. Food Chem. (2020) 68: 1691-1697). Even the highly insoluble corn protein (zein) becomes soluble at pH 5 and 7 and with significantly improved emulsification properties (YH Yong et al. J. Agric Food Chem. (2006): 54: 6034-6040).

The improved functional properties provided by enzymatic deamidation makes the protein deamidase well suited for a variety of food applications of plant protein containing food products like milk analogues with increased protein content, reduced graininess & grittiness, improved mouthfeel, and barista properties, Similarly solutions for the yogurt analogue segments with improved mouthfeel, texture and hydrocolloid replacement. Protein deamidase has also been suggested to improve the texture of plant-based meat analogues and plant-based eggs (X Liu et al Foods (2022) 11: 440).

Deamidation of plant proteins also have a positive impact on the flavour of proteins. Plant proteins are associated with various hydrophobic off-flavours like lipid oxidation products, e.g., having a beany off-flavour, or saponins, phenolics, and flavonoids giving a bitter off-flavour. Enzymatic deamidation of plant proteins reduces the hydrophobicity of the proteins, which therefore reduces the affinity for the hydrophobic off-flavours. Protein deamidase can therefore be applied to improve the flavour of plant proteins by inclusion of the enzyme in the process for recovery of protein concentrates or isolates, or by treatment of recovered proteins like protein isolates (X Liu et al Foods (2022) 11: 440). Flavour improvement is, e.g., demonstrated for soy (I Suppavorasatit et al J. Agric. Food Chem (2012) 60: 7817-7823).

Application of protein deamidase in protein recovery process like the pea protein recovery process leads to improved recovery yield of the proteins, like when applied in the recovery process leading to legume protein concentrate and isolates (WO 2021049591).

Protein deamidase also have several applications on dairy proteins and dairy based foods. Deamidation of whey lead to a better electrostatic repulsion of the proteins, giving a better thermostability, avoiding undesirable aggregation in whey protein solutions (e.g., in protein fortified beverages) when the protein solution is heat-treated (N Miwa et al J. Agric. Food Chem (2013) 61: 2205-2212). Enzymatic deamidation in skim milk leads to much improved solubility, viscosity and provides a translucent milk drink (N Miwa et al International dairy journal (2010) 20: 393-399). Application of protein deamidase in the yogurt process leads to an improved stabilization, which can e.g. be applied to replace pectin and other hydrocolloids in drinking yogurt.

Protein (glutaminase) deamidase can be applied together with other enzymes including other enzymes modifying or degrading protein. Combinations between protein glutamine deamidase and protein asparagine deamidase can provide a higher degree of deamidation of the proteins and thereby an even better applicational performance. Protein deamidase can be applied together with protein crosslinking enzymes like transglutaminase, where the crosslinking of the protein is modified, partly as the transglutaminase will be prevented from reacting with the glutamines which have been converted to glutamic acid by the deamidase. When a combination of transglutaminase and protein deamidase is used in the yogurt process, a texturing effect is obtained, applicable to replace added dairy proteins or hydrocolloids, providing a yogurt with a smooth texture and avoiding the lumpy texture which is seen when transglutaminase is used alone. A similar effect is observed when this enzyme combination is used for production of plant-based yogurt analogues. Furthermore, the protein deamidase can be applied together with proteases where the resulting protein hydrolysate will have improved solubility and taste and changed functional properties.

**Fermentation Broth Formulations or Cell Compositions**

The present invention also relates to a fermentation broth formulation or a cell composition comprising a polypeptide of the present invention. The fermentation broth formulation or the cell composition further comprises additional ingredients used in the fermentation process, such as, for example, cells (including, the host cells containing the gene encoding the polypeptide of the present invention which are used to produce the polypeptide of interest), cell debris, biomass, fermentation media and/or fermentation products. In some embodiments, the composition is a cell-killed whole broth containing organic acid(s), killed cells and/or cell debris, and culture medium.

The term "fermentation broth" as used herein refers to a preparation produced by cellular fermentation that undergoes no or minimal recovery and/or purification. For example, fermentation broths are produced when microbial cultures are grown to saturation, incubated under carbon-limiting conditions to allow protein synthesis (e.g., expression of enzymes by host cells) and secretion into cell culture medium. The fermentation broth can contain unfractionated or fractionated contents of the fermentation materials derived at the end of the fermentation. Typically, the fermentation broth is unfractionated and comprises the spent culture medium and cell debris present after the microbial cells (e.g., filamentous fungal cells) are removed, e.g., by centrifugation. In some embodiments, the fermentation broth contains spent cell culture medium, extracellular enzymes, and viable and/or nonviable microbial cells.

In some embodiments, the fermentation broth formulation or the cell composition comprises a first organic acid component comprising at least one 1-5 carbon organic acid and/or a salt thereof and a second organic acid component comprising at least one 6 or more carbon organic acid and/or a salt thereof. In some embodiments, the first organic acid component is acetic acid, formic acid, propionic acid, a salt thereof, or a mixture of two or more of the foregoing and the second organic acid component is benzoic acid, cyclohexanecarboxylic acid, 4-methylvaleric acid, phenylacetic acid, a salt thereof, or a mixture of two or more of the foregoing.

In one aspect, the composition contains an organic acid(s), and optionally further contains killed cells and/or cell debris. In some embodiments, the killed cells and/or cell debris are removed from a cell-killed whole broth to provide a composition that is free of these components.

The fermentation broth formulation or cell composition may further comprise a preservative and/or anti-microbial (e.g., bacteriostatic) agent, including, but not limited to, sorbitol, sodium chloride, potassium sorbate, and others known in the art.

The cell-killed whole broth or cell composition may contain the unfractionated contents of the fermentation materials derived at the end of the fermentation. Typically, the cell-killed whole broth or cell composition contains the spent culture medium and cell debris present after the microbial cells (e.g., filamentous fungal cells) are grown to saturation, incubated under carbon-limiting conditions to allow protein synthesis. In some embodiments, the cell-killed whole broth or cell composition contains the spent cell culture medium, extracellular enzymes, and killed filamentous fungal cells. In some embodiments, the microbial cells present in the cell-killed whole broth or composition can be permeabilized and/or lysed using methods known in the art.

A whole broth or cell composition as described herein is typically a liquid, but may contain insoluble components, such as killed cells, cell debris, culture media components, and/or insoluble enzyme(s). In some embodiments, insoluble components may be removed to provide a clarified liquid composition.

The whole broth formulations and cell compositions of the present invention may be produced by a method described in WO 90/15861 or WO 2010/096673.

The invention is further defined by the following numbered embodiments:

Embodiment 1. A recombinant fusion polypeptide comprising or consisting of

(a) a first polypeptide comprising a deamidase inhibitory domain, and
(b) a second polypeptide having deamidase activity;

wherein the fusion polypeptide has a thermal unfolding temperature in the range of 65-82°C; and the fusion polypeptide has less than 50% deamidase activity as compared to the second polypeptide.

Embodiment 2. The fusion polypeptide of embodiment 1, wherein the thermal unfolding temperature is in the range of 65-80°C.

Embodiment 3. The fusion polypeptide of embodiment 1, wherein the thermal unfolding temperature is in the range of 67-80°C.

Embodiment 4. The fusion polypeptide of embodiment 1, wherein the thermal unfolding temperature is in the range of 70-80°C.

Embodiment 5. The fusion polypeptide of any of the preceding embodiments, wherein the thermal unfolding temperature is nanoDSF thermal unfolding temperature.

Embodiment 6. The fusion polypeptide of any of the preceding embodiments, wherein the thermal unfolding temperature is nanoDSF thermal unfolding temperature, as described in Example 2.

Embodiment 7. The fusion polypeptide of any of the preceding embodiments, wherein the first polypeptide is located in proximity of the N-terminal end of the fusion polyeptide.

Embodiment 8. The fusion polypeptide of any of the preceding embodiments, wherein the N-terminal end of the first polypeptide is located within 30 amino acids, such as within 20 amino acids or within 10 amino acids, from the N-terminal end of the fusion polyeptide.

Embodiment 9. The fusion polypeptide of any of the preceding embodiments, wherein the second polypeptide is located in proximity of the C-terminal end of the fusion polyeptide.

Embodiment 10. The fusion polypeptide of any of the preceding embodiments, wherein the C-terminal end of the second polypeptide is located within 30 amino acids, such as within 20 amino acids or within 10 amino acids, from the C-terminal end of the fusion polyeptide.

Embodiment 11. The fusion polypeptide of any of the preceding embodiments, wherein the C-terminal end of the first polypeptide is located before the N-terminal end of the second polypeptide.

Embodiment 12. The fusion polypeptide of any of the preceding embodiments, which has less than 40% deamidase activity as compared to the second polypeptide (leakage activity).

Embodiment 13. The fusion polypeptide of any of the preceding embodiments, which has less than 35% deamidase activity as compared to the second polypeptide (leakage activity).

Embodiment 14. The fusion polypeptide of any of the preceding embodiments, wherein the first polypeptide comprises the amino acid sequence motif of SEQ ID NO: 24.

Embodiment 15. The fusion polypeptide of any of the preceding embodiments, wherein the first polypeptide comprises the amino acid sequence motif of SEQ ID NO: 25.

Embodiment 16. The fusion polypeptide of any of the preceding embodiments, wherein the second polypeptide comprises an amino acid sequence motif selected from the group consisting of SEQ ID NO: 21, SEQ ID NO: 22, SEQ ID NO: 23, and combinations thereof.

Embodiment 17. The fusion polypeptide of any of the preceding embodiments, wherein the first polypeptide comprises an amino acid change in a position corresponding to a position selected from the group consisting of 23, 38, 45, 94, and 99, of SEQ ID NO: 2.

Embodiment 18. The fusion polypeptide of any of the preceding embodiments, wherein the first polypeptide comprises an amino acid change in a position corresponding to a position selected from the group consisting of V23, F38, Y45, Y94, and F99, of SEQ ID NO: 2.

Embodiment 19. The fusion polypeptide of any of the preceding embodiments, wherein the first polypeptide comprises an amino acid change in a position corresponding to a position selected from the group consisting of V23S, F38C, Y45G, Y45T, Y94P, F99A, F99G, and F99K, of SEQ ID NO: 2.

Embodiment 19. The fusion polypeptide of any of the preceding embodiments, which has at least 60%, but less than 100%, amino acid sequence identity to an amino acid sequence selected from the group consisting of SEQ ID NO: 5, SEQ ID NO: 10, SEQ ID NO: 15, and SEQ ID NO: 20.

Embodiment 20. The fusion polypeptide of any of the preceding embodiments, which has at least 70%, but less than 100%, amino acid sequence identity to an amino acid sequence selected from the group consisting of SEQ ID NO: 5, SEQ ID NO: 10, SEQ ID NO: 15, and SEQ ID NO: 20.

Embodiment 21. The fusion polypeptide of any of the preceding embodiments, which has at least 80%, but less than 100%, amino acid sequence identity to an amino acid sequence selected from the group consisting of SEQ ID NO: 5, SEQ ID NO: 10, SEQ ID NO: 15, and SEQ ID NO: 20.

Embodiment 22. The fusion polypeptide of any of the preceding embodiments, which has at least 90%, but less than 100%, amino acid sequence identity to an amino acid sequence selected from the group consisting of SEQ ID NO: 5, SEQ ID NO: 10, SEQ ID NO: 15, and SEQ ID NO: 20.

Embodiment 23. The fusion polypeptide of any of the preceding embodiments, which has at least 95%, but less than 100%, amino acid sequence identity to an amino acid sequence selected from the group consisting of SEQ ID NO: 5, SEQ ID NO: 10, SEQ ID NO: 15, and SEQ ID NO: 20.

Embodiment 24. The fusion polypeptide of any of the preceding embodiments, which has at least 96%, at least 97%, at least 98%, or at least 99%, but less than 100%, amino acid sequence identity to an amino acid sequence selected from the group consisting of SEQ ID NO: 5, SEQ ID NO: 10, SEQ ID NO: 15, and SEQ ID NO: 20.

Embodiment 25. The fusion polypeptide of any of the preceding embodiments, which has 1-30 alterations (e.g., substitutions, deletions and/or insertions), preferably 1-20 alterations, 1-10 alterations, or 1-5 alterations, in particular substitutions, as compared to an amino acid sequence selected from the group consisting of SEQ ID NO: 5, SEQ ID NO: 10, SEQ ID NO: 15, and SEQ ID NO: 20.

Embodiment 26. The fusion polypeptide of any of the preceding embodiments, wherein the first polypeptide has at least 60%, but less than 100%, amino acid sequence identity to an amino acid sequence selected from the group consisting of SEQ ID NO: 2, SEQ ID NO: 7, SEQ ID NO: 12, and SEQ ID NO: 17.

Embodiment 27. The fusion polypeptide of any of the preceding embodiments, wherein the first polypeptide has at least 70%, but less than 100%, amino acid sequence identity to an amino acid sequence selected from the group consisting of SEQ ID NO: 2, SEQ ID NO: 7, SEQ ID NO: 12, and SEQ ID NO: 17.

Embodiment 28. The fusion polypeptide of any of the preceding embodiments, wherein the first polypeptide has at least 80%, but less than 100%, amino acid sequence identity to an amino acid sequence selected from the group consisting of SEQ ID NO: 2, SEQ ID NO: 7, SEQ ID NO: 12, and SEQ ID NO: 17.

Embodiment 29. The fusion polypeptide of any of the preceding embodiments, wherein the first polypeptide has at least 90%, but less than 100%, amino acid sequence identity to an amino acid sequence selected from the group consisting of SEQ ID NO: 2, SEQ ID NO: 7, SEQ ID NO: 12, and SEQ ID NO: 17.

Embodiment 30. The fusion polypeptide of any of the preceding embodiments, wherein the first polypeptide has at least 95%, but less than 100%, amino acid sequence identity to an amino acid sequence selected from the group consisting of SEQ ID NO: 2, SEQ ID NO: 7, SEQ ID NO: 12, and SEQ ID NO: 17.

Embodiment 31. The fusion polypeptide of any of the preceding embodiments, wherein the first polypeptide has at least 96%, at least 97%, at least 98%, or at least 99%, but less than 100%, amino acid sequence identity to an amino acid sequence selected from the group consisting of SEQ ID NO: 2, SEQ ID NO: 7, SEQ ID NO: 12, and SEQ ID NO: 17.

Embodiment 32. The fusion polypeptide of any of the preceding embodiments, wherein the second polypeptide has 1-30 alterations (e.g., substitutions, deletions and/or insertions), preferably 1-20 alterations, 1-10 alterations, or 1-5 alterations, in particular substitutions, as compared to an amino acid sequence selected from the group consisting of SEQ ID NO: 4, SEQ ID NO: 9, SEQ ID NO: 14, and SEQ ID NO: 19.

Embodiment 33. The fusion polypeptide of any of the preceding embodiments, wherein the second polypeptide has at least 60% amino acid sequence identity to an amino acid sequence selected from the group consisting of SEQ ID NO: 4, SEQ ID NO: 9, SEQ ID NO: 14, and SEQ ID NO: 19.

Embodiment 34. The fusion polypeptide of any of the preceding embodiments, wherein the second polypeptide has at least 70% amino acid sequence identity to an amino acid sequence selected from the group consisting of SEQ ID NO: 4, SEQ ID NO: 9, SEQ ID NO: 14, and SEQ ID NO: 19.

Embodiment 35. The fusion polypeptide of any of the preceding embodiments, wherein the second polypeptide has at least 80% amino acid sequence identity to an amino acid sequence selected from the group consisting of SEQ ID NO: 4, SEQ ID NO: 9, SEQ ID NO: 14, and SEQ ID NO: 19.

Embodiment 36. The fusion polypeptide of any of the preceding embodiments, wherein the second polypeptide has at least 90% amino acid sequence identity to an amino acid sequence selected from the group consisting of SEQ ID NO: 4, SEQ ID NO: 9, SEQ ID NO: 14, and SEQ ID NO: 19.

Embodiment 37. The fusion polypeptide of any of the preceding embodiments, wherein the second polypeptide has at least 95% amino acid sequence identity to an amino acid sequence selected from the group consisting of SEQ ID NO: 4, SEQ ID NO: 9, SEQ ID NO: 14, and SEQ ID NO: 19.

Embodiment 38. The fusion polypeptide of any of the preceding embodiments, wherein the second polypeptide has at least 96%, at least 97%, at least 98%, or at least 99% amino acid sequence identity to an amino acid sequence selected from the group consisting of SEQ ID NO: 4, SEQ ID NO: 9, SEQ ID NO: 14, and SEQ ID NO: 19.

Embodiment 39. The fusion polypeptide of any of the preceding embodiments, wherein the second polypeptide has up to 30 alterations (e.g., substitutions, deletions and/or insertions), preferably up to 20 alterations, up to 10 alterations, or up to 5 alterations, in particular substitutions, as compared to an amino acid sequence selected from the group consisting of SEQ ID NO: 4, SEQ ID NO: 9, SEQ ID NO: 14, and SEQ ID NO: 19.

Embodiment 40. The fusion polypeptide of any of the preceding embodiments, wherein the first polypeptide comprises a cleavage site for a site-specific endopeptidase, such as glutamyl endopeptidase or trypsin.

Embodiment 41. The fusion polypeptide of any of the preceding embodiments, wherein the first polypeptide comprises a cleavage site for a site-specific endopeptidase, such as glutamyl endopeptidase or trypsin, within 20 amino acids of the C-terminal end.

Embodiment 42. The fusion polypeptide of any of the preceding embodiments, wherein the first polypeptide comprises a cleavage site for a site-specific endopeptidase, such as glutamyl endopeptidase or trypsin, within 15 amino acids of the C-terminal end.

Embodiment 43. The fusion polypeptide of any of the preceding embodiments, wherein the first polypeptide comprises a cleavage site for a site-specific endopeptidase, such as glutamyl endopeptidase or trypsin, within 10 amino acids of the C-terminal end.

Embodiment 44. A polynucleotide encoding the fusion polypeptide of any of the preceding embodiments.

Embodiment 45. The polynucleotide of embodiment 44, which is isolated and/or purified.

Embodiment 46. A nucleic acid construct or expression vector comprising the polynucleotide of embodiment 44, wherein the polynucleotide is operably linked to one or more control sequences that direct the production of the fusion polypeptide in an expression host.

Embodiment 47. A recombinant host cell comprising the nucleic acid construct or expression vector of embodiment 46.

Embodiment 48. The recombinant host cell of embodiment 47, wherein the fusion polypeptide is heterologous to the recombinant host cell.

Embodiment 49. The recombinant host cell of any of embodiments 47 to 48, wherein at least one of the one or more control sequences is heterologous to the polynucleotide encoding the fusion polypeptide.

Embodiment 50. The recombinant host cell of any of embodiments 47 to 49, which comprises at least two copies, e.g., three, four, or five, or more copies of the polynucleotide of any one of embodiments 44 to 46.

Embodiment 51. The recombinant host cell of any of embodiments 47 to 50, which is a yeast recombinant host cell, *e.g.*, a *Candida*, *Hansenula*, *Kluyveromyces*, *Pichia*, *Saccharomyces*, *Schizosaccharomyces*, or *Yarrowia* cell, such as a *Kluyveromyces lactis*, *Saccharomyces carlsbergensis*, *Saccharomyces cerevisiae*, *Saccharomyces diastaticus*, *Saccharomyces douglasii*, *Saccharomyces kluyveri*, *Saccharomyces norbensis*, *Saccharomyces oviformis*, or *Yar-*

*rowia lipolytica* cell.

Embodiment 52. The recombinant host cell of any of embodiments 47 to 50, which is a filamentous fungal recombinant host cell, *e.g.*, an *Acremonium*, *Aspergillus*, *Aureobasidium*, *Bjerkandera*, *Ceriporiopsis*, *Chrysosporium*, *Coprinus*, *Coriolus*, *Cryptococcus*, *Filibasidium*, *Fusarium*, *Humicola*, *Magnaporthe*, *Mucor*, *Myceliophthora*, *Neocallimastix*, *Neurospora*, *Paecilomyces*, *Penicillium*, *Phanerochaete*, *Phlebia*, *Piromyces*, *Pleurotus*, *Schizophyllum*, *Talaromyces*, *Thermoascus*, *Thielavia*, *Tolypocladium*, *Trametes*, or *Trichoderma* cell, in particular, an *Aspergillus awamori*, *Aspergillus foetidus*, *Aspergillus fumigatus*, *Aspergillus japonicus*, *Aspergillus nidulans*, *Aspergillus niger*, *Aspergillus oryzae*, *Bjerkandera adusta*, *Ceriporiopsis aneirina*, *Ceriporiopsis caregiea*, *Ceriporiopsis gilvescens*, *Ceriporiopsis pannocinta*, *Ceriporiopsis rivulosa*, *Ceriporiopsis subrufa*, *Ceriporiopsis subvermispora*, *Chrysosporium inops*, *Chrysosporium keratinophilum*, *Chrysosporium lucknowense*, *Chrysosporium merdarium*, *Chrysosporium pannicola*, *Chrysosporium queenslandicum*, *Chrysosporium tropicum*, *Chrysosporium zonatum*, *Coprinus cinereus*, *Coriolus hirsutus*, *Fusarium bactridioides*, *Fusarium cerealis*, *Fusarium crookwellense*, *Fusarium culmorum*, *Fusarium graminearum*, *Fusarium graminum*, *Fusarium heterosporum*, *Fusarium negundi*, *Fusarium oxysporum*, *Fusarium reticulatum*, *Fusarium roseum*, *Fusarium sambucinum*, *Fusarium sarcochroum*, *Fusarium sporotrichioides*, *Fusarium sulphureum*, *Fusarium torulosum*, *Fusarium trichothecioides*, *Fusarium venenatum*, *Humicola insolens*, *Humicola lanuginosa*, *Mucor miehei*, *Myceliophthora thermophila*, *Neurospora crassa*, *Penicillium purpurogenum*, *Phanerochaete chrysosporium*, *Phlebia radiata*, *Pleurotus eryngii*, *Talaromyces emersonii*, *Thielavia terrestris*, *Trametes villosa*, *Trametes versicolor*, *Trichoderma harzianum*, *Trichoderma koningii*, *Trichoderma longibrachiatum*, *Trichoderma reesei*, or *Trichoderma viride* cell.

Embodiment 53. The recombinant host cell of any of embodiments 47 to 50, which is a prokaryotic recombinant host cell, e.g., a Gram-positive cell selected from the group consisting of *Bacillus*, *Clostridium*, *Enterococcus*, *Geobacillus*, *Lactobacillus*, *Lactococcus*, *Oceanobacillus*, *Staphylococcus*, *Streptococcus*, or *Streptomyces* cells, or a Gram-negative bacteria selected from the group consisting of *Campylobacter*, *E. coli*, *Flavobacterium*, *Fusobacterium*, *Helicobacter*, *Ilyobacter*, *Neisseria*, *Pseudomonas*, *Salmonella*, and *Ureaplasma* cells, such as *Bacillus alkalophilus*, *Bacillus amyloliquefaciens*, *Bacillus brevis*, *Bacillus circulans*, *Bacillus clausii*, *Bacillus coagulans*, *Bacillus firmus*, *Bacillus lautus*, *Bacillus lentus*, *Bacillus licheniformis*, *Bacillus megaterium*, *Bacillus pumilus*, *Bacillus stearothermophilus*, *Bacillus subtilis*, *Bacillus thuringiensis*, *Streptococcus equisimilis*, *Streptococcus pyogenes*, *Streptococcus uberis*, and *Streptococcus equi* subsp. *Zooepidemicus*, *Streptomyces achromogenes*, *Streptomyces avermitilis*, *Streptomyces coelicolor*, *Streptomyces griseus*, and *Streptomyces lividans* cells.

Embodiment 54. The recombinant host cell of any of embodiments 47 to 50, which is a *Bacillus licheniformis* cell.

Embodiment 55. The recombinant host cell of any of embodiments 47 to 54, which is isolated.

Embodiment 56. The recombinant host cell of any of embodiments 47 to 55, which is purified.

Embodiment 57. A method of producing the fusion polypeptide of any of embodiments 1 to 37, comprising cultivating the recombinant host cell of any of embodiments 41 to 50 under conditions conducive for production of the fusion polypeptide.

Embodiment 58. The method of embodiment 51, further comprising recovering the fusion polypeptide.

Embodiment 59. A method for producing a polypeptide having deamidase activity, comprising contacting the fusion polypeptide of any of embodiments 1 to 43 with a site-specific endopeptidase to separate the first polypeptide from the second polypeptide.

Embodiment 60. The method of embodiment 59, wherein the site-specific endopeptidase is selected from the group consisting of glutamyl endopeptidase, trypsin-like endopeptidases, and chymotrypsin-like endopeptidases.

Embodiment 61. A composition exhibiting deamidase activity, comprising the first polypeptide and the second polypeptide of the fusion polypeptide of any of embodiments 1 to 43, wherein the first and second polypeptides are not covalently linked.

Embodiment 62. The composition of embodiment 61, which further comprises a site-specific endopeptidase.

Embodiment 63. The composition of embodiment 61 or 62, which is a liquid composition comprising

(a) 0.001-25% w/w of the first polypeptide,
(b) 0.001-25% w/w of the second polypeptide,
(c) polyol(s), and
(d) water.

Embodiment 64. A method for modifying a protein, comprising contacting the protein with the composition of any of embodiments 61 to 63.

Embodiment 65. The method of embodiment 64, wherein the modification is a deamidation of a glutamine residue.

The present invention is further described by the following examples that should not be construed as limiting the scope of the invention.

**EXAMPLES**

**Strains**

The *Chryseobacterium* sp-62563 strain was isolated from a soil sample collected in Sibhult, Sweden in September 2013.

**EXAMPLE 1**

**Deamidase activity**

Materials

Glutamyl endopeptidase from *Bacillus licheniformis*

FITC-PP-Dnp (FITC-Ahx-His-His-Gln-Ser-Ser-ED-Dnp) was a custom synthesized substrate molecule from TAG Copenhagen, Kong Georgs Vej 12, DK-2000 Frederiksberg (www.tagc.com), where:

FITC is fluorescein with excitation and emission maxima at approx. 490 nm and 520 nm,
Ahx is Aminohexanoic acid,
ED is ethylene diamine, and
Dnp is 2,4-dinitrophenyl (fluorescein fluorescence quencher).

Deamidase maturation assay

The assay describes maturation of the deamidase pro-form (cleavage of the propeptide and deamidase domains) and measures the relative activity of the pro-form ("native sample") and the active deamidase ("activated sample") after treatment with a site-specific endopeptidase. The site-specific endopeptidase used in the assay is a glutamyl endopeptidase from *Bacillus licheniformis.*

After purification and quantification, as described in Examples 6 and 7, the deamidase (pro-form) sample was split into two samples. One sample was normalized to 50nM deamidase in 100mM HEPES buffer, pH 7.0, 0.01% v/v Triton X detergent and labeled "native". The second sample was normalized to 50nM deamidase in 100mM HEPES buffer, pH 7.0, 0.01% v/v Triton X detergent, including 10μg/mL of glutamyl endopeptidase and labelled "activated". Both samples were incubated 30 minutes at room temperature.

After incubation, 50μL of both samples were transferred to a standard black 96 well plate and were added

20μL of 0.25μg/mL FITC-PP-Dnp
50μL of 50μg/mL glutamyl endopeptidase
130μL of 100mM HEPES buffer pH 7.0, and
0.01% v/v Triton X detergent.

A Biotek Synergy H1 fluorescence plate reader was used to measure the fluorescence signal (RFU) for 30 min using emission/excitation wavelengths 485 nm/525 nm.

Data was analyzed for initial rate using variable time intervals depending on the shape of curve (signal vs time). The initial rate for each sample was normalized relative to the initial rate of a fully activated deamidase reference enzyme (produced by the wildtype donor strain). The activity was measured as "% initial rate", i.e., % initial rate of sample molecule relative to initial rate of mature deamidase reference enzyme.

Deamidase activity of fusion polypeptide (Leakage activity)

The assay measures the activity of the deamidase pro-form (fusion polypeptide), denoted "native"; relative to the maturated form, denoted "activated". This ratio is also referred to as "Leakage activity". The maturation was carried out by pre-treatment with a glutamyl endopeptidase from *Bacillus licheniformis.*

After purification and quantification, as described in Examples 6 and 7, the deamidase (pro-form) sample was split into two halves, and one half ("activated") was normalized to 10nM deamidase in 100mM HEPES buffer, pH7.0, 0.01% v/v Triton X detergent, and 10μg/mL glutamyl endopeptidase was added.

The other half ("native") was normalized to a concentration of 100nM deamidase in 100mM HEPES buffer, pH7.0, 0.01% v/v Triton X detergent.

Both the "native" and "activated" samples were reacted with 0.2 μg/mL FITC-PP-Dnp at 30°C under gentle shaking for 90 minutes. After reaction, 100μL of each of the two samples were heated to 95°C for 5 min in a PCR thermocycler to inactivate the deamidase activity. After inactivation, 50μL heat-treated samples were transferred to a standard black 96 well plate and 150μL of 13 μg/mL glutamyl endopeptidase was added to hydrolyse the fraction of FITC-PP-Dnp where Glutamine was converted to Glutamate by deamidase activity.

Endpoint signal was measured in a standard platereader using emission/excitation wavelengths 485 nm/525 nm in a Biotek Synergy H1 fluorescence plate reader.

Leakage activity was defined as activity (RFU/nM deamidase) of the deamidase pro-form ("native") relative to the activity of the maturated deamidase ("activated") in percent.

## EXAMPLE 2

### Nano differential scanning fluorimetry (nanoDSF) - thermal unfolding temperature

Nano differential scanning fluorimetry (nanoDSF) was used to evaluate the conformational stability of the fusion polypeptides of the invention. The molecules were exposed to a temperature gradient, as indicated below. The resulting changes in structure is reflected in changes in fluorescence intensity and gives a measure of temperature stability. Binding of the propeptide domain to the deamidase domain contributes to stability of the molecule, thus the nanoDSF thermal unfolding temperatures also give information on the propeptide-deamidase binding affinity.

His-Tag purified samples were received in an elution buffer from an IMAC (Immobilized Metal Affinity Chromatography) column; 20mM sodium phosphate; 500mM sodium chloride; 500mM imidazole; pH 7.4.

60 μL sample was transferred in replicates to a black-bottomed 384 well plate, and the plate was briefly centrifugated to remove potential air bubbles.

The thermal unfolding temperature of the samples was analyzed using a Prometheus NT.Plex system from NanoTemper Technologies GmbH, with the following settings:

(i) Temperature scan rate: 3.3°C per minute; and
(ii) Temperature scan interval: 20-95°C.

During the run, samples were loaded in capillaries (Prometheus NT.Plex - Capillary Chips, Standard, Cat# PR-AC002) before being subjected to the temperature gradient. The generated data were analyzed by the PR.Stability analysis v.1.0.1 software. Midpoint unfolding temperatures ($T_m$, °C) were annotated based on the first derivative of the 330 nm trace. In some cases, more than one $T_m$ may be observed. In these cases, the main peak in the first derivative trace at 330 nm was chosen as the $T_m$ of the sample.

## EXAMPLE 3

### Thermal unfolding temperature of wildtype fusion polypeptides

Using the nanoDSF procedure, as described in Example 2, we have measured the thermal unfolding temperature of some recombinant wildtype fusion polypeptides that comprise a deamidase inhibitory domain and a deamidase domain. They all exhibit very low deamidase activity and cannot be activated/maturated by proteolytic cleavage/separation of the two domains. The sequence identity of the exemplified polypeptides shows that they represent a high sequence diversity.

Table 1. NanoDSF thermal unfolding temperatures of wildtype fusion polypeptides.

| Donor organism | Sequence | Amino acid sequence identity | NanoDSF thermal unfolding temperature |
|---|---|---|---|
| *Chryseobacterium* sp-62563 | SEQ ID NO: 5 | 100% | 85.3°C |
| *Chryseobacterium gambrini* | SEQ ID NO: 10 | 85% | 89.3°C |
| *Chryseobacterium culicis* | SEQ ID NO: 15 | 95% | 85.2°C |
| *Chryseobacterium defluvii* | SEQ ID NO: 20 | 74% | 89.0°C |

The data in Table 1 shows that wildtype fusion polypeptides with quite different amino acid sequences have high thermal unfolding temperatures (as determined by nanoDSF), indicating a high stability and binding affinity between the propeptide and the deamidase domain.

## EXAMPLE 4

**Thermal unfolding temperatures of deamidase variant fusion polypeptides**

Using the nanoDSF procedure, as described in Example 2, we have measured the thermal unfolding temperature of some recombinant fusion polypeptides. The recombinant fusion polypeptides all comprise a variant deamidase propeptide (first polypeptide), which is derived from the propeptide/donor sequence of *Chryseobacterium* sp-62563 (SEQ ID NO: 2).

All recombinant fusion polypeptides exhibited sufficiently low deamidase activity to allow recombinant expression/production (see Example 8) and could also be maturated by proteolytic cleavage and separation of the fusion polypeptide (see Example 9). The variant deamidase propeptides comprised the mutations as shown in Table 2, and the nanoDSF thermal unfolding temperature of the resulting fusion polypeptides is also shown in Table 2.

Table 2. NanoDSF thermal unfolding temperatures of variant fusion polypeptides.

| Donor organism | Donor sequence | Variant amino acid substitution | NanoDSF thermal unfolding temperature |
|---|---|---|---|
| *Chryseobacterium* sp-62563 | SEQ ID NO: 2 | V23S | 69.8°C |
| *Chryseobacterium* sp-62563 | SEQ ID NO: 2 | F38C | 76.2°C |
| *Chryseobacterium* sp-62563 | SEQ ID NO: 2 | Y45G | 65.2°C |
| *Chryseobacterium* sp-62563 | SEQ ID NO: 2 | Y45T | 73.7°C |
| *Chryseobacterium* sp-62563 | SEQ ID NO: 2 | Y94P | 75.2°C |
| *Chryseobacterium* sp-62563 | SEQ ID NO: 2 | F99A | 76.4°C |
| *Chryseobacterium* sp-62563 | SEQ ID NO: 2 | F99G | 74.1°C |
| *Chryseobacterium* sp-62563 | SEQ ID NO: 2 | F99K | 75.4°C |

The data in Table 2 show that by introducing a mutation in the deamidase inhibitory domain, the binding affinity/stability of the fusion polypeptide comprising the deamidase propeptide (and deamidase inhibitory domain) and the deamidase is reduced enough to allow maturation by cleavage/separation of the two domains. At the same time, the variant fusion polypeptide exhibits sufficiently low deamidase activity to allow recombinant expression without compromising viability of the recombinant host cell.

## EXAMPLE 5

**Identification and cloning of protein glutamine deamidases**

Identification of the protein glutamine deamidase gene from *Chryseobacterium sp-62563*

A protein glutamine deamidase encoding DNA sequence (SEQ ID NO: 1 fused to SEQ ID NO: 3) was isolated from a bacterial strain sampled in Sweden, as described above. The donor strain's taxonomy was established by 16S ribosomal DNA sequencing. Blast homology analysis using 16S ribosomal sequence returned 99.1% homology with 16S DNA sequence from EMBL:KC108937 (*Chryseobacterium sp. UA-JF4202*) and 99.1% with 16S DNA sequence from EMBL:AM988898 (*Chryseobacterium sp. AKB-2008-HE85*). The strain was therefore given a new name of *Chryseobacterium sp-62563.*

*Chryseobacterium sp-62563* genome sequencing was conducted on pure genomic DNA using Next Generation Sequencing IIlumina technology, where sequence reads were assembled with ibda v1.1.1 (Bioinformatics. 2012 Jun 1;28(11):1420-8.) and GeneFinder Prodigal 2.50 (BMC Bioinformatics. 2010; 11: 119.) was used to annotate open reading frames. A protein glutamine deamidase DNA sequence (SEQ ID NO: 1 fused to SEQ ID NO: 3) was identified within this assembly. This gene encodes a protein glutamine deamidase pro-form (SEQ ID NO: 5), which consists of a propeptide domain (SEQ ID NO: 2) and a deamidase domain (SEQ ID NO: 4).

The gene encoding the protein glutamine deamidase pro-form (SEQ ID NO: 5) was PCR-amplified from genomic DNA of the *Chryseobacterium sp-62563,* where the native N-terminal region encompassing a native lipoprotein signal peptide (Teufel et al., "SignalP 6.0 predicts all five types of signal peptides using protein language models", Nature Biotechnology (2022)) was replaced with a *Bacillus licheniformis* alpha amylase secretion signal (amyL signal peptide as described in WO 2014206806). A 6His tag was added at the C-terminal end of the CDS to ease enzyme recovery. This engineered sequence was fused to the transcriptional promoter from sequence GENESEQN:BGM50663 (WO 2015004013) and to the transcriptional terminator from sequence GENESEQN:BET98406 (WO 2018009520) by linear overlapping PCR using SOE-PCR fusion strategy (Horton, R. M., et al. (1989) Engineering hybrid genes without the use of restriction enzymes: gene splicing by overlap extension Gene 77 (1) 61-68). The SOE PCR method is also described in more detail in patent application WO 2003095658. Additional extra 5' and 3' DNA regions corresponding to the L-arabinosse (ara) insertion locus were added during the SOE-PCR reaction.

A low protease background *Bacillus subtilis* strain derived from B. *subtilis A164* (ATCC 6051A) was used for expression of the *Chryseobacterium* sp-62563 protein glutamine deamidase. Integration of the SOE PCR products into the expression host genome was done by homologous recombination into the *Bacillus subtilis* chromosomal region of the L-arabinose (ara) operon and selection driven with the erm (erythromycin) marker on agar plates.

Production strategy of the deamidase variants

Point mutation variants were generated using the giga-primer strategy described in WO 2016066756 with non-degenerate primers. In a first PCR (PCR1), the C-terminal fragment was generated using a mutagenic forward primer and a reverse primer complementary to a sequence necessary for homologous integration in *Bacillus* genome. In a second PCR, the C-terminal fragment from PCR1 was used as giga-primer and a second primer complementary to a sequence necessary for homologous integration into the Bacillus genome was used. Resulting variants were spread on agar plates with erythromycin and single colonies picked for sequence verification before fermentation. The polymerase used for the PCR reaction was Phusion DNA polymerase (Finnzymes Oy (ThermoFisher Scientific)).

Cloning and expression of deamidase from *Chryseobacterium gambrini*, *Chryseobacterium culitis* and *Chryseobacterium defluvii*

Public sequences TREMBL:A0A2S9CPY3 from *Chryseobacterium culicis,* TREMBL:A0A1N7P918 from *Chryseobacterium gambrini* and TREMBL:A0A495SBZ2 from *Chryseobacterium defluvii* were engineered to ensure a similar expression strategy as for the *Chryseobacterium sp-62563* deamidase SEQ ID NO: 5. The N-terminal region of the wild-type sequences (TREMBL:A0A2S9CPY3 from *Chryseobacterium culicis,* TREMBL:A0A1N7P918 from *Chryseobacterium gambrini* and TREMBL:A0A495SBZ2 from *Chryseobacterium defluvii*) including their lipoprotein signal peptides were replaced by *Bacillus licheniformis* alpha amylase secretion signal as described above. The linker region (defined by sequence-alignment with SEQ ID NO: 5) located at the C-terminal end of the propeptide region (SEQ ID NO: 2) and the N-terminal end of the deamidase peptide (SEQ ID NO: 4) was replaced by the linker region of *Chryseobacterium sp-62563,* and a 6 Histidine tag was added to the C-terminal to ensure a similar process for expression maturation and purification as for the *Chryseobacterium sp-*62563 deamidase (SEQ ID NO: 5). Engineered sequences corresponding to SEQ ID NO: 10 for *Chryseobacterium culitis* deamidase (encoded by propeptide domain SEQ ID NO: 6 and deamidase domain SEQ ID NO: 8), SEQ ID NO: 15 for *Chryseobacterium gambrini* deamidase (encoded by propeptide domain SEQ ID NO: 11 and deamidase domain SEQ ID NO: 13), and SEQ ID NO: 20 for *Chryseobacterium defluvii* deamidase (encoded by propeptide domain SEQ ID NO: 16 and deamidase domain SEQ ID NO: 18) were purchased as synthetic DNAs. 5' and 3' overlaps corresponding to the amyl(3A) signal peptide on the 5'end and HisTag/stop/terminator in the 3' end were also included to allow generating constructs for transformation by linear overlapping PCR using SOE-PCR fusion strategy (Horton, R. M., et al. (1989) Engineering hybrid genes without the use of restriction enzymes: gene splicing by overlap extension Gene 77 (1) 61-68). SOE PCR method is also described in more detail in patent application WO 2003095658.

A low protease background *Bacillus subtilis* strain derived from B. *subtilis A164* (ATCC 6051A) was used for expression of the three *Chryseobacterium* protein glutamine deamidase pro-forms: SEQ ID NO: 10, SEQ ID NO: 15, and SEQ ID NO: 20. Integration of the SOE PCR products into the expression host genome was done by homologous recombination into the *Bacillus subtilis* chromosomal region of the L-arabinose (ara) operon and selection driven with the erm (erythromycin) marker on agar plates.

**EXAMPLE 6**

**Fermentation and purification of protein glutamine deamidase**

Sequence-verified *Bacillus subtilis* transformed with constructs encompassing SEQ ID NO: 1 fused to SEQ ID NO: 3, SEQ ID NO: 6 fused to SEQ ID NO: 8, SEQ ID NO: 11 fused to SEQ ID NO: 13, SEQ ID NO: 16 fused to SEQ ID NO: 18, or their corresponding point-mutation variants were inoculated into 96-well plates filled beforehand with 1mL Cal18-2 media supplemented with erythromycin (Cal18-2 media composition is described in EP1187925B1) and fermentation was carried out at 30°C under 700rpm agitation. After 4 days, plates were centrifuged to pellet *Bacillus* cells, and 0.4mL supernatant was transferred to 96-well His MultiTrap purification filters (GE Healthcare 11-0036-62 AB). His tag purification was performed according to manufacturer's protocol. Elution of protein glutamine deamidase SEQ ID NO: 5, 10, 15, 20 and protein glutamine deamidase variants was made in 0.2mL buffer composed of 20mM sodium phosphate, 0.5M NaCl, 500mM imidazole pH7.4. For activation assays, His purified samples were buffer-exchanged using 96-well desalting PD MultiTrap G-25 (Cytiva) and elution was made in 50mM Hepes, 100mM NaCl pH7 buffer.

**EXAMPLE 7**

**Expression analysis and quantification of protein glutamine deamidase**

His tag purified samples were analyzed by SDS PAGE electrophoresis and separated on 26-wells NuPAGE™ gels (Invitrogen ref. WG1403Bx10). 10μL purified enzymes were mixed with 10μL sample buffer mix (containing loading buffer and reducing agent) cooked at 99°C for 2min. and 10μL were loaded on gel. Electrophoresis was performed using 1x MOPS buffer and after electrophoresis, gels were colored in Ready Blue™ reagent (Sigma Aldrich) overnight. Gels were scanned on a BioRad Criterion Stain Free™ imagery system.

Tiff-files format scans from SDS PAGE gels were used for densitometry analysis carried out with ImageJ software v1.52a. Band intensity of the deamidase pro-form (around 32kDa) were measured and normalized across gels using wild type deamidase proform as standard. Densitometric measurements were used to evaluate impact of the mutations on expression level of the protein glutamine deamidase.

**EXAMPLE 8**

**Leakage activity of recombinant fusion polypeptides**

Using the Leakage activity procedure, as described in Example 1, we have measured the activity of some recombinant fusion polypeptides as compared to the corresponding maturated/active deamidase. The recombinant fusion polypeptides all comprise a variant deamidase propeptide (first polypeptide), which is derived from the propeptide/donor sequence of *Chryseobacterium* sp-62563 (SEQ ID NO: 2).

The variant deamidase propeptides comprised the mutations as shown in Table 3, and the measured leakage activity of the resulting recombinant fusion polypeptides is also shown in Table 3.

Table 3. Leakage activity of recombinant fusion polypeptides.

| Donor organism | Donor sequence | Variant amino acid substitution | Leakage activity |
|---|---|---|---|
| *Chryseobacterium* sp-62563 | SEQ ID NO: 2 | V23S | 10% |
| *Chryseobacterium* sp-62563 | SEQ ID NO: 2 | F38C | 26% |
| *Chryseobacterium* sp-62563 | SEQ ID NO: 2 | Y45G | 2% |
| *Chryseobacterium* sp-62563 | SEQ ID NO: 2 | Y45T | 2% |
| *Chryseobacterium* sp-62563 | SEQ ID NO: 2 | Y94P | 23% |
| *Chryseobacterium* sp-62563 | SEQ ID NO: 2 | F99A | 20% |
| *Chryseobacterium* sp-62563 | SEQ ID NO: 2 | F99G | 20% |
| *Chryseobacterium* sp-62563 | SEQ ID NO: 2 | F99K | 32% |

The data in Table 3 show that the recombinant fusion polypeptides all exhibited less than 35% deamidase activity as compared to the mature deamidase (leakage activity).

### EXAMPLE 9

**Maturation of deamidase from recombinant fusion polypeptides**

Using the deamidase maturation procedure, as described in Example 1, we have measured the activity of some maturated/activated deamidases. The recombinant fusion polypeptides, from which the maturated deamidases were prepared, all comprise a variant deamidase propeptide (first polypeptide), which is derived from the propeptide/donor sequence of *Chryseobacterium* sp-62563 (SEQ ID NO: 2). The deamidase (second polypeptide) is the same for all the recombinant fusion polypeptides and also originate from *Chryseobacterium* sp-62563 (SEQ ID NO: 4).

All the recombinant fusion polypeptides exhibited sufficiently low deamidase activity (leakage activity, see Example 8) to allow recombinant expression/production. The variant deamidase propeptides comprised the mutations as shown in Table 4, and the relative activity of the resulting maturated deamidases is also shown in Table 4.

Table 4. Relative activity of maturated deamidase.

| Donor organism | Donor sequence | Variant amino acid substitution | Relative activity of "activated" sample (% initial rate) |
|---|---|---|---|
| *Chryseobacterium* sp-62563 | SEQ ID NO: 2 | V23S | ≥ 100% |
| *Chryseobacterium* sp-62563 | SEQ ID NO: 2 | F38C | ≥ 100% |
| *Chryseobacterium* sp-62563 | SEQ ID NO: 2 | Y45G | ≥ 100% |
| *Chryseobacterium* sp-62563 | SEQ ID NO: 2 | Y45T | ≥ 100% |
| *Chryseobacterium* sp-62563 | SEQ ID NO: 2 | Y94P | ≥ 100% |
| *Chryseobacterium* sp-62563 | SEQ ID NO: 2 | F99A | ≥ 100% |
| *Chryseobacterium* sp-62563 | SEQ ID NO: 2 | F99G | ≥ 100% |
| *Chryseobacterium* sp-62563 | SEQ ID NO: 2 | F99K | ≥ 100% |

The data in Table 4 show that the recombinant fusion polypeptides were excellently maturated to release deamidase activity, using a site-specific endopeptidase.

## Claims

1. A recombinant fusion polypeptide comprising or consisting of

    (a) a first polypeptide comprising a deamidase inhibitory domain, and
    (b) a second polypeptide having deamidase activity;

    wherein the fusion polypeptide has a thermal unfolding temperature in the range of 65-82°C; and the fusion polypeptide has less than 50% deamidase activity as compared to the second polypeptide.

2. The recombinant fusion polypeptide of claim 1, wherein the C-terminal end of the first polypeptide is located before the N-terminal end of the second polypeptide.

3. The recombinant fusion polypeptide of any of the preceding claims, wherein the first polypeptide comprises the amino acid sequence motif of SEQ ID NO: 24 and/or the amino acid sequence motif of SEQ ID NO: 25.

4. The recombinant fusion polypeptide of any of the preceding claims, wherein the second polypeptide comprises an amino acid sequence motif selected from the group consisting of SEQ ID NO: 21, SEQ ID NO: 22, SEQ ID NO: 23, and combinations thereof.

5. The recombinant fusion polypeptide of any of the preceding claims, wherein the fusion polypeptide has at least 80%, at least 90%, at least 95%, at least 96%, at least 97%, at least 98%, or at least 99%, but less than 100%, amino acid sequence identity to an amino acid sequence selected from the group consisting of SEQ ID NO: 5, SEQ ID NO: 10, SEQ ID NO: 15, and SEQ ID NO: 20.

6. The recombinant fusion polypeptide of any of the preceding claims, wherein the first polypeptide has at least 80%, at least 90%, at least 95%, at least 96%, at least 97%, at least 98%, or at least 99%, but less than 100%, amino acid sequence identity to an amino acid sequence selected from the group consisting of SEQ ID NO: 2, SEQ ID NO: 7, SEQ ID NO: 12, and SEQ ID NO: 17.

7. The recombinant fusion polypeptide of any of the preceding claims, wherein the second polypeptide has at least 80%, at least 90%, at least 95%, at least 96%, at least 97%, at least 98%, or at least 99% amino acid sequence identity to an amino acid sequence selected from the group consisting of SEQ ID NO: 4, SEQ ID NO: 9, SEQ ID NO: 14, and SEQ ID NO: 19.

8. The recombinant fusion polypeptide of any of the preceding claims, wherein the first polypeptide comprises an amino acid change in a position corresponding to a position selected from the group consisting of 23, 38, 45, 94, and 99, of SEQ ID NO: 2; preferably the amino acid change is a substitution.

9. A method of producing the fusion polypeptide of any of the preceding claims, comprising cultivating a recombinant host cell comprising a polynucleotide encoding the recombinant fusion polypeptide, under conditions conducive for production of the recombinant fusion polypeptide.

10. A method for producing a polypeptide having deamidase activity, comprising contacting the recombinant fusion polypeptide of any of claims 1-9 with a site-specific endopeptidase to separate the first polypeptide from the second polypeptide.

11. A composition exhibiting deamidase activity, comprising the first polypeptide and the second polypeptide of the recombinant fusion polypeptide of any of claims 1-9, wherein the first and second polypeptides are not covalently linked.

12. The composition of claim 11, which further comprises a site-specific endopeptidase.

13. The composition of claim 11 or 12, which is a liquid composition comprising

    (a) 0.001-25% w/w of the first polypeptide,
    (b) 0.001-25% w/w of the second polypeptide,
    (c) polyol(s), and
    (d) water.

14. A method for modifying a protein, comprising contacting the protein with the composition of any of claim 11-13; preferably the modification is a deamidation of a glutamine residue.

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YIN XINXIN ET AL: "Combinatorial engineering for efficient production of protein-glutaminase in Bacillus subtilis", ENZYME AND MICROBIAL TECHNOLOGY, STONEHAM, MA, US, vol. 150, 29 June 2021 (2021-06-29), XP086765364, ISSN: 0141-0229, DOI: 10.1016/J.ENZMICTEC.2021.109863 [retrieved on 2021-06-29] * the whole document * | 1-4,7-12 | INV. C12N9/80 |
| X | -& DATABASE UniProt [Online] 1 June 2001 (2001-06-01), "SubName: Full=Protein-glutaminase {ECO:0000313¦EMBL:BAB21508.1}; Flags: Precursor;", XP055949920, retrieved from EBI accession no. UNIPROT:Q9AQQ8 Database accession no. Q9AQQ8 * the whole document * | 1-4,7,8 | |
| X | DATABASE UniProt [Online] "RecName: Full=Gln_deamidase_2 domain-containing protein {ECO:0000259¦Pfam:PF18626};", XP002807290, retrieved from EBI accession no. UNIPROT:A0A521ADJ3 Database accession no. A0A521ADJ3 * the whole document * | 1-8 | |
| A | CN 111 434 770 A (UNIV EAST CHINA NORMAL; TAIXING DONGSHENG BIO TECH CO LTD) 21 July 2020 (2020-07-21) * the whole document * | 1-14 | |

**TECHNICAL FIELDS SEARCHED (IPC)**

C12N

The present search report has been drawn up for all claims

| Place of search | Date of completion of the search | Examiner |
|---|---|---|
| Munich | 8 August 2022 | Strobel, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 0942

08-08-2022

| Patent document cited in search report | | Publication date | Patent family member(s) | Publication date |
|---|---|---|---|---|
| CN 111434770 | A | 21-07-2020 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9517413 A
- WO 9522625 A
- US 5223409 A
- WO 9206204 A
- WO 9425612 A
- WO 2017144177 A
- EP 238023 A
- WO 0001793 A
- WO 2006034710 A
- WO 9932595 A
- GB 1483591 A
- WO 9307263 A
- WO 9723606 A
- WO 9739116 A
- US 4016040 A
- US 4713245 A
- US 4106991 A
- EP 170360 A
- EP 304332 A
- EP 304331 A
- WO 9009440 A
- WO 9009428 A
- US 4661452 A
- WO 2013188331 A
- EP 238216 A
- WO 2014123466 A
- WO 2021049591 A
- WO 9015861 A
- WO 2010096673 A
- WO 2014206806 A
- WO 2015004013 A
- WO 2018009520 A
- WO 2003095658 A
- WO 2016066756 A
- EP 1187925 B1

### Non-patent literature cited in the description

- **HASHIZUME et al.** Crystal structures of protein glutaminase and its pro forms converted into enzyme-substrate complex. *Journal of Biological Chemistry,* vol. 286 (44), 38691-38702
- **COOPER et al.** *EMBO J.,* 1993, vol. 12, 2575-2583
- **DAWSON et al.** *Science,* 1994, vol. 266, 776-779
- **MARTIN et al.** *J. Ind. Microbiol. Biotechnol.,* 2003, vol. 3, 568-576
- **SVETINA et al.** *J. Biotechnol.,* 2000, vol. 76, 245-251
- **RASMUSSEN-WILSON et al.** *Appl. Environ. Microbiol.,* 1997, vol. 63, 3488-3493
- **WARD et al.** *Biotechnology,* 1995, vol. 13, 498-503
- **CONTRERAS et al.** *Biotechnology,* 1991, vol. 9, 378-381
- **EATON et al.** *Biochemistry,* 1986, vol. 25, 505-512
- **COLLINS-RACIE et al.** *Biotechnology,* 1995, vol. 13, 982-987
- **CARTER et al.** *Proteins: Structure, Function, and Genetics,* 1989, vol. 6, 240-248
- **STEVENS.** *Drug Discovery World,* 2003, vol. 4, 35-48
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite*
- **K. HUYNH ; C.L. PARTCH.** Analysis of protein stability and ligand interactions by thermal shift assay. *Current Protocols in Protein Science,* 2015
- **R. LONESCU ; L. SHI.** Proteins: Thermal Unfolding. *Encyclopedia of Industrial Biotechnology,* 2009
- **CUNNINGHAM ; WELLS.** *Science,* 1989, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- **JUMPER et al.** Highly accurate protein structure prediction with AlphaFold. *Nature,* 2021, vol. 596, 583-589

- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **DAVIS et al.** Basic Methods in Molecular Biology. Elsevier, 2012
- **FORD et al.** *Protein Expression and Purification,* 1991, vol. 2, 95-107
- **SAMBROOK et al.** Molecular Cloning: A Laboratory Manual. Cold Spring Harbor Lab, 1989
- **SONG et al.** *PLOS One,* 2016, vol. 11 (7), e0158447
- **MUKHERJEE et al.** *Trichoderma: Biology and Applications,* 2013
- **SCHMOLL ; DATTENBÖCK.** Gene Expression Systems in Fungi: Advancements and Applications. *Fungal Biology,* 2016
- **SMOLKE et al.** *Synthetic Biology: Parts, Devices and Applications,* 2018
- **ROMANOS et al.** *Yeast,* 1992, vol. 8, 423-488
- **HUE et al.** *J. Bacteriol.,* 1995, vol. 177, 3465-3471
- **GEISBERG et al.** *Cell,* 2014, vol. 156 (4), 812-824
- **MOROZOV et al.** *Eukaryotic Cell,* 2006, vol. 5 (11), 1838-1846
- **HAMBRAEUS et al.** *Microbiology,* 2000, vol. 146 (12), 3051-3059
- **KABERDIN ; BLÄSI.** *FEMS Microbiol. Rev.,* 2006, vol. 30 (6), 967-979
- **GUO ; SHERMAN.** *Mol. Cellular Biol.,* 1995, vol. 15, 5983-5990
- **FREUDL.** *Microbial Cell Factories,* 2018, vol. 17, 52
- **XU et al.** *Biotechnology Letters,* 2018, vol. 40, 949-955
- **SESHASAYEE et al.** *Subcellular Biochemistry,* 2011, vol. 52, 7-23
- **BALLEZA et al.** *FEMS Microbiol. Rev.,* 2009, vol. 33 (1), 133-151
- **PATEL ; GUPTA.** *Int. J. Syst. Evol. Microbiol.,* 2020, vol. 70, 406-438
- **HEINZE et al.** *BMC Microbiology,* 2018, vol. 18, 56
- **BURKE et al.** *Proc. Natl. Acad. Sci. USA,* 2001, vol. 98, 6289-6294
- **CHOI et al.** *J. Microbiol. Methods,* 2006, vol. 64, 391-397
- **DONALD et al.** *J. Bacteriol.,* 2013, vol. 195 (11), 2612-2620
- **HAWKSWORTH et al.** Ainsworth and Bisby's Dictionary of The Fungi. CAB International, University Press, 1995
- **LI et al.** *Microbial Cell Factories,* 2017, vol. 16, 168
- **YELTON et al.** *Proc. Natl. Acad. Sci. USA,* 1984, vol. 81, 1470-1474
- **CHRISTENSEN et al.** *Bio/Technology,* 1988, vol. 6, 1419-1422
- **LUBERTOZZI ; KEASLING.** *Biotechn. Advances,* 2009, vol. 27, 53-75
- Biology and Activities of Yeast. Soc. App. Bacteriol. Symposium Series. 1980
- **WINGFIELD.** *Current Protocols in Protein Science,* 2015, vol. 80 (1), 611-6135
- **LABROU.** *Protein Downstream Processing,* 2014, vol. 1129, 3-10
- Particle size enlargement. **C. E. CAPES ; 1980.** Handbook of Powder Technology. Elsevier, vol. 1
- Powdered detergents. Surfactant Science Series. Marcel Dekker, 1998, vol. 71, 140-142
- Powdered detergent. Surfactant Science Series. Marcel Dekker, 1998, vol. 71, 140-142
- Principles of Powder Technology. John Wiley & Sons, 1990
- **Z-I JIANG.** *J Cereal Science,* 2015, vol. 64, 126-132
- **I SUPPAVORASATIT et al.** *J. Agric. Food Chem,* 2011, vol. 59, 11621-11628
- **L FANG et al.** *J, Agric. Food Chem.,* 2020, vol. 68, 1691-1697
- **YH YONG et al.** *J. Agric Food Chem.,* 2006, vol. 54, 6034-6040
- **X LIU et al.** *Foods,* 2022, vol. 11, 440
- **I SUPPAVORASATIT et al.** *J. Agric. Food Chem,* 2012, vol. 60, 7817-7823
- **N MIWA et al.** *J. Agric. Food Chem,* 2013, vol. 61, 2205-2212
- **N MIWA et al.** *International dairy journal,* 2010, vol. 20, 393-399
- *Bioinformatics,* 01 June 2012, vol. 28 (11), 1420-8
- *BMC Bioinformatics,* 2010, vol. 11, 119
- **TEUFEL et al.** SignalP 6.0 predicts all five types of signal peptides using protein language models. *Nature Biotechnology,* 2022
- *Gene,* vol. 77 (1), 61-68
- **HORTON, R. M. et al.** Engineering hybrid genes without the use of restriction enzymes: gene splicing by overlap extension. *Gene,* 1989, vol. 77 (1), 61-68